(19) **Europäisches Patentamt / European Patent Office / Office européen des brevets**

(11) **EP 3 187 201 B2**

(12) # NEW EUROPEAN PATENT SPECIFICATION
After opposition procedure

(45) Date of publication and mention
of the opposition decision:
**20.07.2022 Bulletin 2022/29**

(45) Mention of the grant of the patent:
**29.05.2019 Bulletin 2019/22**

(21) Application number: **15203108.4**

(22) Date of filing: **30.12.2015**

(51) International Patent Classification (IPC):
*A61M 1/00* (2006.01)     *A61B 5/021* (2006.01)
*A61B 5/0404* (2006.01)   *A61B 5/0476* (2006.01)
*A61B 5/11* (2006.01)     *A61B 5/024* (2006.01)
*A61B 5/00* (2006.01)     *A61M 5/24* (2006.01)
*A61M 5/142* (2006.01)    *A61N 1/04* (2006.01)
*A61N 1/365* (2006.01)    *G01P 1/02* (2006.01)
*G01P 15/18* (2013.01)    *G01P 15/02* (2013.01)

(52) Cooperative Patent Classification (CPC):
**A61B 5/68; A61B 5/74; A61M 1/73; A61M 1/90;**
**A61M 5/14244; A61N 1/0484; A61N 1/36542;**
A61B 5/02141; A61B 5/02438; A61B 5/1123;
A61B 5/332; A61B 5/369; A61B 2560/0276;
A61M 2005/2418; A61M 2209/088

(54) **PORTABLE MEDICAL DEVICE**

TRAGBARE MEDIZINISCHE VORRICHTUNG

DISPOSITIF MÉDICAL PORTABLE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**05.07.2017 Bulletin 2017/27**

(73) Proprietor: **Paul Hartmann AG**
**89522 Heidenheim (DE)**

(72) Inventors:
• **Klusmann, Johanna**
**89522 Heidenheim (DE)**

• **Beyrle, Karina**
**89522 Heidenheim (DE)**
• **Eckstein, Axel**
**89522 Heidenheim (DE)**
• **Hofstetter, Jürgen**
**89522 Heidenheim (DE)**
• **Croizat, Pierre**
**89522 Heidenheim (DE)**

(56) References cited:
**EP-A1- 2 345 893      US-A1- 2015 040 665**
**US-A1- 2015 073 338**

## Description

### FIELD OF THE INVENTION

The invention relates to the field of portable medical devices adapted to be carried by a user. In particular, the invention relates to the use of sensor technology for monitoring integrity and for improving operational reliability of such portable medical devices.

### BACKGROUND TO THE INVENTION

Portable medical devices, which exert a therapeutic and/or a diagnostic function on a patient are continuously gaining in importance, because they improve mobility of the user (i.e. the patient). Improved mobility leads to an increased quality of life for the patient. Also, portable medical devices may expand the field of application for some type of devices: Whereas previously the use of complex medical treatment appliances was mostly restricted to a hospital environment, the same type of device (though in a more compact format) may now be used by physicians in private practice as well as in outpatient care.

The above-mentioned expression "portable medical device" means that the patient can carry the device along so that he/she is mobile and can nevertheless be permanently treated or monitored, i.e. without interruption. An expression used with an identical meaning is "ambulatory medical device". The portable (or ambulatory) device may thereby be held on the body of the patient and be carried along by means of any fastening means, for example in the form of a flexible belt or a shoulder strap. A portable device of the above-mentioned type may naturally also be used for stationary operation, i.e. detached from the body of the patient. In this case, it may e.g. be mounted to a hospital bed or be deposited next to the hospital bed.

Preferred examples of portable medical devices adapted to be carried by a user include a negative pressure wound therapy device, an ambulatory deep brain stimulation device, an ambulatory cardiac stimulator, an ambulatory infusion device, in particular an insulin pump, an ambulatory blood pressure unit, an ambulatory pulse monitor, an ambulatory electrocardiogram device, an ambulatory electroencephalogram device or an ambulatory diagnosis system for determining metabolites circulating in the blood.

US-20150040665-A1 discloses a device for detecting impacts to a body part of a user. Such impacts can occur, for example, during sport or other physical activity. The device comprises at least two sensors.

US-20150073338-A1 discloses an enteral feeding pump having a sensor for detecting movement of the pump.

EP-2345893-A1 discloses a blood-sugar level detection device, which can also measure movement information associated with human body activity. Movement is determined using an acceleration sensor. Correlations between blood-sugar levels and living activity may help, for example, to improve measurement accuracy.

A specific non-limiting example for a portable medical device is a vacuum wound treatment apparatus. Vacuum treatment of wounds, also commonly called "negative pressure wound therapy", has become a main therapeutic approach for the treatment of severe and/or chronic wounds. Vacuum wound treatment devices have been described many times, in particular, in US 2004/0073151 A1, WO 2009/047524 A2, EP 1 905 465 A1, WO 2008/039314 A2 or EP 777 504 81 as well as in EP 1 863 549 B1, EP 2 464 394 A1, WO 2012/156174 A1 or EP 2 464 393 A1 of the assignee. In devices of this type for vacuum treatment of wounds, a suction pump (sometimes incorrectly called "vacuum pump") communicates with the wound or the wound area via a suction line, wherein a wound dressing with an air-tight cover material is provided for air-tight sealing of the wound and the wound area, such that a vacuum can be generated in the wound region and fluids can be extracted by suction from the wound region. The term vacuum in connection with the present invention defines an air pressure that is lower than the ambient air pressure (atmospheric air pressure), in particular, inside a wound dressing. The cover material of a wound dressing for air-tight sealing of a wound region must therefore be designed in such a fashion that it withstands the pressure difference that is established such that a vacuum can actually be applied to and maintained in the wound region. The wound dressing and the cover material are, however, typically flexible to a certain degree. In the field of vacuum therapy for the treatment of wounds, the vacuum is quantitatively defined as the pressure difference between ambient air pressure and the air pressure applied below the cover material. In the field of vacuum therapy, this pressure difference is typically at most 250 mmHg (mm mercury column) (1 mm Hg=1 Torr =133.322 Pa). This vacuum range of up to maximally 250 mmHg has turned out to be suitable for wound healing. A preferred vacuum range is between 10 and 150 mmHg. For typical vacuum treatment, the vacuum that is applied to the wound using the device can either be kept substantially constant with time or can be varied with time, in particular, in cycles, which can be realized by a correspondingly designed and programmed control device for the vacuum-generating device, in particular, in dependence on further parameters. An advantageously flexible suction line, e.g. in the form of a drainage hose, is provided for applying a vacuum and advantageously also for extracting body fluids, the drainage hose communicating at one end with the wound area or the wound region via a so-called port in the area of the wound cover material, and at the other end communicating with a container for receiving the sucked body fluids, or with the vacuum generating device.

In contrast to a stationary medical device, a portable medical treatment device may frequently be exposed to a changing environment. The device can be used even outside of a medical facility, for example during travel or in a home environment. Removing the device from the comparatively well-protected environment of a medical facility increases the likelihood that the device may, for example, fall to the ground or crash against any objects. Such drops or crashes may come along with very high acceleration and deformation forces acting on the components of the device. Some of these impact events may - under some circumstances - lead to an immediate failure of the apparatus. Such a complete malfunction will easily be noticed by the user. Also, even minor malfunctions can usually be detected by an electronic self-control system, which many up-to-date medical devices are provided with. The electronic self-control system will inform the user about the malfunction and optionally terminate therapy.

Other impacts events however do not lead to any immediate malfunction of the device but instead leads to hidden damages of electronic and/or mechanical components of the medical device. Such hidden damages may lead to unpredictable operational disorders or even complete breakdowns in the future. Any of these potential future malfunctions may lead to a sudden interruption of the therapy, which can possibly cause harm to the patient.

Moreover, there are also impacts, which do not cause any harm to the device, even though the device had been exposed to considerable acceleration and deformation forces.

Microelectronic sensor technology including acceleration sensors has been used to monitor the integrity of medical apparatuses including mobile medical devices. US 2008 0276684 for example describes the use of one or more sensors to detect environmental (external) influences on the operability of a medical device, such as an optical tracking system. In particular, US 2008 0276684 aims at detecting external influences, which might affect calibration of the device.

Similarly, EP 1 867 955, describes the use of sensors to detect the external influences, such as temperature, crash, acceleration, tension, pressure or magnetic fields, which might interfere with calibration or functionality of the device. Sensor data may be recorded and stored. The method for detecting environmental impacts described in EP 1 867 955 may optionally include the use of predetermined thresholds, which serve to identify such impacts, which supposedly have impaired proper calibration of the instrument.

It is an inherent drawback of such sensor technology based monitoring systems that in some cases warnings are generated, although no actual physical damage is present. A false warning may disconcert the user. In the worst case the control system of the device terminates therapy (after detecting an impact of a certain strength) although no damage is present.

Based on a portable medical apparatus of the type having a microelectronic impact sensor for detecting acceleration, it is the underlying purpose of the present invention to further optimize the user friendliness and operational safety of the device. The medical device should be as reliable and as failproof as possible. Ideally, even a technically less skilled user or patient should be given the feeling that she/he can safely control operation. It is another purpose of the present invention to avoid false alarms.

We have found that a portable medical device according to claim 1, wherein the controller is adapted to execute a classification algorithm on impact sensor data sets, may lead to improved reliability and operational safety of the device. The classification algorithm proposed herewith allows to discriminate a first impact sensor data set, said first impact sensor data set being correlated to an impact event which is detrimental for the medical device, from a second impact sensor data set, said second impact sensor data set being correlated to an impact event, which is not detrimental for the medical device. As a side-effect, it was found that classification of impact sensor data sets can also be used by the development engineers to identify and analyse mechanical strain, to which a portable medical device is typically exposed outside of a hospital environment. This information, which is - for example - collected during the service of the device, can be used to improve stability and reliability of future devices.

SUMMARY OF THE INVENTION

The invention is defined in the appended claims.
Execution of the classification algorithm allows for a more reliable discrimination between impacts, which are detrimental for the device and those impacts, which are not detrimental for the apparatus.
Using the classification algorithm, the controller can identify an impact, which may lead to operational malfunction in the future, even if no visible damages are present. The user can be warned and will be invited to let the device check by a service technician. The frequency of any unforeseen interruptions of the therapy or diagnosis can be reduced. Although the occurrence of false alarms cannot be eliminated completely the frequency of false alarms based on the impact sensors is reduced significantly. In general, the reliability of the device can be improved.

DETAILED DESCRIPTION OF THE INVENTION

During inspection of malfunctioning portable negative pressure wound therapy devices (i.e. a portable medical device, which is particularly preferred to be used in connection with the present invention), the inventors of the present invention found, that at least 50 % of the technical defects or malfunctions observed were most likely related to external impacts that were acting on the devices. These presumptive harmful impacts included crashes, exposure to humidity or moisture and extreme temperatures. In most such cases no signs of impact were visible when inspecting the outside of the housing of the devices. The vast majority of those impacts (more than 90 % of all impacts) were crashes. In many cases these crashes did not leave any visible traces on the housing of the device (i.e. the damage was not visible when inspecting the outside of the housing of the devices). However, the crashes resulted in loosened electronic contacts or untightened mechanical connections, such as tube connections. In many cases an impact of that type was the reason for a future malfunction. The present invention provides means and methods to classify different types of impact acting on a portable medical device by executing a classification algorithm. In particular, the classification algorithm allows to allocate an individual impact event either to a first class of impact events or to a second class of impact events. The first class of impact events ("detrimental impacts") includes impact events which lead to an immediate malfunction of the portable medical device. The first class of impact events also includes impact events which carry the potential of generating a malfunction at a later time. The second class of impact events ("not detrimental impacts") includes impact events, which are not causing any immediate and/or future damages. The classification algorithm is executed by a microelectronic controller. The algorithm allows to discriminate between the first and the second class of impact events, without generating an unacceptable high number of false discriminations.

In the context of the present invention, the expression "impact event, which is detrimental for the device" means that the impact event is usually or typically detrimental for the device (first class of impacts). In practice, there may be individual impact events classified as "impact event, which is detrimental for the device", which do however not lead to any actual damages of the device. Nevertheless, if the impact event falls into the first class of impacts described as "detrimental for the device", the impact correlated to the event at least has the capability of causing damages even if an actual damage does not appear. Accordingly, the expression "impact event, which is not detrimental for the device" means that the impact event is usually or typically not detrimental for the device, even if such an event may nevertheless cause damages to the device in some rare cases.

The at least one electrically actuated means for performing a therapeutic and/or a diagnostic function on a patient may for example include (but are not limited to) a suction pump for draining body fluids, an infusion pump, a pressure sensor for monitoring blood pressure, an electroencephalographic recording unit or an electrochemical measuring unit capable of determining metabolite concentrations in human blood in vivo.

Aptly, the at least one mobile electric power supply may include a rechargeable accumulator or a battery. The power supply is required to power the electric components of the device such as the microelectronic controller or the impact sensor.

The portable medical device according to the invention comprises a control unit having at least one microelectronic controller and at least one electronic memory. The control unit is the central instance for controlling, monitoring and operating function of the device. The control unit comprises a user interface for the user, such as a touch-screen display, adapted to receive user settings and/or to display one or more system conditions. The electronic memory is another component of the control unit. In genera!, the electronic memory is required for storing operating instructions for the microelectronic controller and/or for recording status information related to the function of the device. Moreover, an electronic memory is also required to store any impact sensor related data (i.e. impact sensor raw data, processed impact sensor data or information related to the result of an analysis of impact sensor data sets).

The device further comprises at least one microelectronic multi-channel impact sensor unit, wherein the multi-channel impact sensor unit is adapted to detect linear acceleration ($a_x$; $a_y$; $a_z$) along the three space axes (x; y; z) and angular rates ($\omega_x$; $\omega_y$; $\omega_z$) around the three space axes (x; y; z). Detection of linear acceleration ($a_x$; $a_y$; $a_z$) along the three space axes (x; y; z) and angular rates ($\omega_x$; $\omega_y$; $\omega_z$) around the three space axes (x; y; z) is done simultaneously. The multi-channel impact sensor unit comprises at least three acceleration sensor functions adapted to detect linear acceleration ($a_x$; $a_y$; $a_z$) along each of the three space axes (x; y; z) and at least three gyration sensor functions adapted to detect angular rates ($\omega_x$; $\omega_y$; $\omega_z$) around each of the three space axes (x; y; z). Accordingly, the multi-channel impact sensor generates sensor data related to the three linear acceleration values and to the three angular rate values. A single impact event leads to a series of impact sensor data received from each of the six channels of the sensor impact sensor unit (three channels for linear acceleration and three channels for angular rates) over the time of recording. In the context with the present invention the entirety of impact sensor data (i.e. originating from the six output channels as a whole) related to a single impact event is called "impact sensor data set".

The impact sensor unit has an interface, which is required to electronically transfer the impact sensor data sets to the microelectronic controller.

Preferably the multi-channel impact sensor unit comprises a microelectronic inertial measurement unit (abbreviated: "IMU") for detection of linear acceleration and rotation, whereby the microelectronic inertial measurement unit (IMU) is integrated in a single chip.

The electric components of the device such as the electrically actuated means for performing a therapeutic and/or a diagnostic function, the power supply, the control unit and the at least one impact sensor unit are contained in a housing.

The at least one multi-channel impact sensor unit interacts with at least one microelectronic controller and/or with at least one electronic memory, both of which are a component of the control unit. Interaction of the impact sensor unit with the microelectronic controller may optionally include processing of the raw data produced by the impact sensor unit and selection of the data to be recorded on an electronic memory. According to the invention, the microelectronic controller executes a classification algorithm. The impact sensor data sets, which are generated as a consequence of an impact acting on the device, constitute the input for the classification algorithm (the microelectronic controller executing the classification algorithm may receive the impact sensor data sets either directly from the impact sensor or alternatively from the memory). The output of the classification algorithm is an allocation of an impact data set corresponding to a single impact event into one of two classes, i.e. detrimental impact (first class) a not detrimental impact (second class). Classification of impact data sets is done using standard classification algorithm known in the art. Complexity of data analysis can be reduced, if (for each single impact event) only the maximum acceleration/rotation values from each of the channels are processed.

Classification algorithms may be based upon collection of training data sets. Collection of training data sets is done by repeatedly exposing the device to conditions, which belong to each of the two classes (i.e. the first class of impacts, the "detrimental impacts" and the second class of impacts, the "not detrimental impacts") and by storing the impact sensor data sets received form the training events. Accuracy of the classification increases by lifting the number of training events. At least 50, preferably 100, most preferably 500 training experiments for each of the two classes should be performed to obtain a reliable data basis for classification.

The classification algorithm includes a support vector machine (sometimes also shortly called SVM) known from the art. Support vector machines, which have its origin in machine learning, can be used for classification. It includes learning algorithms that analyze data with the objective of recognising patterns. A plurality of training examples, which belong to one of the two classes to be classified, is required. The training algorithm generates a model, which is able to allocate new experiments into one of the two classes. Translated into the object of the present invention, the model is a representation of the single impact events as points or vectors in a space. The single points are mapped in a way, using as less dimensions as possible, so that the two classes are preferably divided by a clear gap, which has a maximum distance to each training data point on both sides (binary linear classifier). In mathematical terms, the support vector machine is used to construct a hyperplane (or a set of hyperplanes in a high-dimensional space), whereby a maximum margin hyperplane is sought. The events to be classified are then entered into that same space. Classification is determined based on which side of the gap (in mathematical terms: on which side of the hyperplane) they appear.

If a linear separation is not possible, support vector machines are able to perform a non-linear classification, for example by using the "Kernel Method", which includes mapping the data points into higher-dimensional spaces.

In general, a hyperplane has one dimension less than the space it is dividing. For the practical purposes, on which the present invention is based upon, it was found that a classification algorithm including a support vector machine using a two-dimensional space and a linear separation line (in this case the hyperplane is a one-dimensional line) is preferred.

It was also found, that it is particularly advantageous to construct the two-dimensional space (a plane) using the following mathematical method:

- The first dimension of the two dimensional space is defined by the "magnitude $M_\alpha$" corresponding to the vector length of the linear acceleration values ($a_x$; $a_y$; $a_z$) measured by the three linear acceleration channels of the multi-channel impact sensor. Magnitude $M_\alpha$ is calculated using the formula:

$$M_\alpha = \sqrt{(a_x^2 + a_y^2 + a_z^2)}$$

Same as for acceleration, the absolute dimension for $M_\alpha$ of is m/s$^2$. For the purposes of the present invention, it is also possible to use a relative unit (i.e. a value without dimensions) for $M_\alpha$. A relative value for $M_\alpha$ can - for example - be generated by entering the value corresponding to the output of each acceleration sensor channel into the aforementioned formula.

Magnitude $M_\alpha$ reflects the overall strength of linear acceleration stress acting on the device. For calculation of magnitude $M_\alpha$ the peak (maximum) acceleration values from each channel related to a single impact event are used.

- The second dimension of the two dimensional space is defined by the "magnitude $M_\omega$" corresponding to the vector length of the angular rate values ($\omega_x$; $\omega_y$; $\omega_z$) measured by the three linear gyration sensor channels of the multi-channel impact sensor. Magnitude $M_\omega$ is calculated using the formula:

$$M_\omega = \sqrt{(\omega_x^2 + \omega_y^2 + \omega_z^2)}$$

Same as for rotation, the absolute dimension for $M_\omega$ is rad/s (the unit dps is used as well; dps stands for degree per second). For the purposes of the present invention, it is also possible to use a relative unit (i.e. a value without dimensions) for $M_\omega$. A relative value

for $M_\omega$ can - for example - be generated by entering the value corresponding to the output of each gyration sensor channel into the aforementioned formula.

Magnitude $M_\omega$ reflects the overall strength of the gyration the device is subjected to. For calculation of magnitude $M_\omega$ the peak (maximum) rotation values from each channel related to a single impact event are used.

According to the invention, the microelectronic controller is adapted to execute a classification algorithm. The method of classification of impact events includes at least the following steps i) to iii):

i) Providing a portable medical device, which comprises

- an electrically actuated means for performing a therapeutic and/or a diagnostic function on a patient,
- at least one mobile electric power supply,
- a control unit having at least one microelectronic controller and at least one electronic memory,
- at least one multi-channel impact sensor unit, wherein the impact sensor unit is adapted to detect linear acceleration ($a_x$; $a_y$; $a_z$) along the three space axes (x; y; z) and angular rates ($\omega_x$; $\omega_y$; $\omega_z$) around the three space axes (x; y; z), wherein the impact sensor unit generates impact sensor data sets, and wherein the data sets generated by the sensor are electronically transferred to the controller via an interface,
- a housing, in which the electric components of the device are contained.

ii) Detecting an impact by means of the multi-channel impact sensor unit.

iii) Executing a classification algorithm, which allows to discriminate a first impact sensor data set, said first impact data set being correlated to a first impact event which is detrimental for the device, from a second impact sensor data set, said second impact data set being correlated to a second impact event which is not detrimental for the device. Said first impact event is a member of the first class of impacts. Said second impact event is a member of the second class of impacts.

Optionally the method further includes recording the result obtained from the classification algorithm and/or displaying an alarm message to the user, if the result obtained from executing the classification algorithm indicates, that an impact event, which is detrimental for the device, has occurred.

According to a preferred embodiment, the invention also includes a method for creating a separation line (hyperplane), which allows to discriminate sensor data sets, which are correlated to an impact event, which is detrimental for the portable medical device from sensor data sets, which are correlated to an impact event, which is not detrimental for the portable medical device, comprising the steps of

i) providing a portable medical device adapted to be carried by a user, said device comprising

- an electrically actuated means for performing a therapeutic and/or a diagnostic function on a patient,
- at least one mobile electric power supply,
- a control unit having at least one microelectronic controller and at least one electronic memory,
- at least one muiti-channel impact sensor unit, wherein the impact sensor unit is adapted to detect linear acceleration ($a_x$; $a_y$; $a_z$) along the three space axes (x; y; z) and angular rates ($\omega_x$; $\omega_y$; $\omega_z$) around the three space axes (x; y; z), wherein the impact sensor unit generates impact sensor data sets, and wherein the data sets generated by the sensor are electronically transferred to the controller via an interface,
- a housing, in which the electric components of the device are contained,

ii) exposing the portable medical device to a series of impact experiments (training experiments), wherein each impact experiment comprises the step

- exposing the device to a plurality of impact events,
- recording the linear acceleration values ($a_x$; $a_y$; $a_z$) along the three space axis (x; y; z) and the angular rates ($\omega_x$; $\omega_y$; $\omega_z$) around the three space axis (x; y; z) occurring during each of the plurality of impact experiment,
- calculating the magnitude $M_\alpha$ and the magnitude $M_\omega$ from the impact sensor data set obtained during each of the plurality of impact experiment, whereby the peak values originating from each single impact event are used,
- entering the magnitude $M_\alpha$ and the magnitude $M_\omega$ into a two-dimensional diagram, thereby creating a data vector specific for each impact experiment,
- analysing the technical condition of the portable medical device after each impact experiment,
- assigning each of the plurality of impact experiments as detrimental for the device or as not detrimental for the device, based on the analysis of the technical condition,

- annotating each data vector to the classification detrimental or not detrimental,

iii) creating a separation line (hyperplane) in the two-dimensional diagram, which separates the data vectors corresponding to a detrimental impact from the data vectors which are corresponding to a not detrimental impact, whereby a maximum-margin hyperplane is chosen.

The expression "maximum-margin hyperplane" stands for a hyperplane, which has the largest distance to the nearest training-data point of any class.

The information obtained by the classification algorithm can advantageously be analysed in combination with the strength of the impact (i.e. the level of acceleration and/or rotation that the device was subjected to).

Advantageously, the information obtained by the classification algorithm can be used to inform a user that a detrimental impact acting on the device has occurred, even if no visible malfunction of the device is present, In this case the user may, for example, be warned, that a possible malfunction related to the impact may occur in the future (even if no malfunction of the device is currently appearing). Advantageously, the device may be locked, if an impact exceeding a certain strength has acted on the device. A locking of the device may be implemented to retain a user from using a device for therapy, which may carry a potential (but not visible) damage related to an external impact.

It is also possible to use the information obtained from the classification algorithm (optionally in combination with the level of acceleration and/or rotation measured by the multi-channel impact sensor unit) for maintenance purposes. In a (non-limiting) example a service technician can use the information to selectively inspect or exchange components, which are typically prone to deficiency when exposed to certain kinds of external acceleration impacts. The technician may, for example, selectively exchange the main control board of the device. He may also check all electric and/or mechanical connections. As known to a person skilled in the art, many other specific maintenance actions can be performed, once the nature and optionally the strength of the external impact has been determined by the impact sensor.

Advantageously, when making use of the present invention, a patient using the device as well as a caregiver (such as a nurse) using the device, will be warned, if an external impact may affect proper functionality and integrity of the treatment device.

It is a particular advantage of the present invention, that impacts, which were acting on the device in the past and which did not leave any visible traces, can be detected and/or recorded.

Preferably, the impact sensor unit comprises a microelectronic inertial measurement unit (IMU), which is integrated in a single electronic chip. One example well suited for this embodiment of the invention is the IMU motion sensor LSM9DS0 ("iNEMO inertial module") from ST Microelectronics, United Kingdom. This inertial module device additionally includes a three cannel (3D) magnetometer, which can be used, in addition to the detection and classification of crash impacts, to determine any magnetic fields acting on the device. It is possible to program the controller to display an alarm message, if a certain magnetic field strength is acting on the device. This is of particular relevance, when using the portable medical device in a hospital environment, where medical devices generating strong magnetic fields (such as an MRT-device) are used. The magnetic fields could harm the electronic components of the portable medical device.

It is also possible to provide the portable medical device with additional sensors adapted to detect any kind of detrimental environmental impacts, such as humidity sensors, temperature sensors, magnetic field sensors (like the one included in the LSM9DS0 module) and atmospheric air pressure sensor.

A device according to the present invention comprises at least one electronic memory, which is adapted to at least store operating instructions for the controller and/or to record status information related to the function of the device.

It is further preferred, that the electronic memory of the portable medical device is adapted to store sensor data generated by the at least one impact sensor and/or to store a result obtained by the classification algorithm. According to this embodiment, one and the same electronic memory component is adapted to store operating instructions for the controller and/or to record status information related to the function of the device as well as any impact sensor related data.

According to another preferred embodiment, the portable medical device is adapted to record the impact sensor data on the at least one electronic memory and/or on any additional electronic storage medium.

According to a more preferred embodiment, the device comprises an additional electronic storage medium, in particular a flash memory, wherein the additional electronic memory is adapted for recording only impact sensor related data. Accordingly, the additional (separate) electronic memory is working independent of the memory, which is used by the control unit to store operating instructions or status information.

According to a very favourable embodiment of the invention, the impact sensor unit is a component of a "stand-alone impact sensor module". The module comprises at least one impact sensor unit and a further microelectronic controller (i.e. in addition to the microelectronic controller, which is a component of the control unit) cooperating with the impact sensor unit. The additional microelectronic controller may also be integrated in the impact sensor unit chip. The module further comprises a data interface, such as a RS232 interface, a memory, such as a flash memory and a real-time clock. The sensor unit, the interface, the memory and the real-time clock are in direct interaction with the additional microelectronic controller (i.e. with the microelectronic controller of the stand-alone impact sensor unit module). A separate battery or accumulator supplies electric energy to the components of the module.

In principle, it is possible to record the complete output of the impact sensor unit (i.e. the raw data). Due to the high sampling rate of a typical impact sensor unit (an acceleration sensor has a typical sampling rate of 1000 Hz), storage of the produced raw data will lead to extensive consumption of memory space. It is therefore preferred, that the raw data are stored for a short period of time only, such as a time window of, for example, 5 seconds, to enable the microelectronic controller to analyse the data including the classification analysis. After analysis, the raw data are overwritten and only the result of the analysis is stored for an extended period of time. According to another preferred method to further save memory space, the storing of impact sensor related data (i.e. impact sensor raw data or information related to the result of an analysis of impact sensor raw data) is done only under the proviso that the strength of the impact is above a predetermined level. A device according to this preferred embodiment is adapted to record an impact, if the impact and/or rotation values measured by at least one of the six sensors channels of the multi-channel impact sensor unit are above a predetermined threshold level. The expression "to record an impact" includes storing the sensor raw data on an electronic memory component, such as a flash memory. The expression "to record an impact" also includes storing any information or results that any algorithm (including but not limited to the classification algorithm) running on a controller deduces from the impact sensor raw data. Saving only the results of an evaluation is the most effective way to save memory. Additional saving of memory space can be achieved by applying standard methods of data compression know in the art. In general, the predetermined threshold for data storage can conveniently be selected at a sufficient distance to the level, which is correlated to the certified tolerance of the device for the specific external impact. The data storage threshold for acceleration should be correlated to the type of device and to the mechanical stability of the device.

It was found, that an adequate data storage impact threshold for linear acceleration is an acceleration value selected from the range of 3 g to 100 g, in particular 5 g to 50 g, most preferred 10 g. It is also possible to use magnitude $M_\alpha$ and/or magnitude $M_\omega$ as a reference parameter for data storage. Magnitudes $M_\alpha$ and $M_\omega$ are calculated as explained above herein. For example, a threshold for magnitudes $M_\alpha$ selected from a value be-

tween 2 m/s$^2$ and 1000 m/s$^2$ can be used. The threshold has to be selected for each type of device individually, taking into consideration the specific nature of the device with regard to stability, form, size, weight and the like.

It is also proposed that the portable medical device includes a real time clock, whereby the device is adapted to record the time of an impact detected by the impact sensor unit. This means that the storing of impact sensor related data (as explained earlier, impact sensor related data include impact sensor raw data or information related to the result of an analysis of impact sensor raw data) is done together with the time derived from the real time clock. According to this embodiment, the sensor related data are provided with a "time stamp". The real time clock is running permanently. It may be powered by a separate power supply, if available.

A device according to the present invention further comprises at least one mobile electric power supply, such as a rechargeable battery, to power any electric functions of the device. In particular, the electric power supply is required to provide electric energy to the electrically actuated means for performing a therapeutic and/or a diagnostic function on a patient, to the microelectronic controller, to the impact sensor unit, to the electronic memory and to a display, if present.

According to a preferred embodiment, it has been found advantageous to provide the at least one impact sensor unit with a separate power supply, in particular a battery, a rechargeable battery or a capacitor, whereby the separate power supply is independent of a power supply supplying the electrically actuated means for performing a therapeutic and/or a diagnostic function on a patient as well as the microelectronic controller.

The separate power supply shall be adapted to safeguard a permanent supply of electric energy to the impact sensor unit, even if the battery required to drive the therapeutic and/or a diagnostic function is empty.

According to another preferred embodiment, the portable medical device is capable of detecting and analysing detrimental impacts, even if the device is switched off. According to this further preferred embodiment, it is possible, for example, to warn a user about potential (though not visible from external inspection) damages of the device that could compromise proper function of the device during therapy. Such a warning could, for example, be displayed as soon as the device is switched on.

A permanent activity of the impact sensor unit according to this embodiment (i.e. even if the main functions of the portable medical device are switched off) leads to permanent consumption of electric energy by the impact sensor unit. A good solution in order to keep power consumption of the impact sensor within acceptable limits is to include a sleep-to-wakeup function. This means that the impact sensor unit is running in a power saving mode, as soon as the portable medical device is switched off. As soon as the main functions of the device are switched on, the impact sensor unit is awake. The same transition from the sleep to the awake modus occurs also, if an impact (such as a crash leading to an acceleration detected by a linear acceleration sensor) above a predetermined wakeup threshold level is detected by the sleeping impact sensor. The power saving mode (during sleeping) is characterised by a lower sampling activity, which requires less electric energy compared to the wake state.

In particular, the portable medical device comprises a sleep-to-wakeup function, the sleep-to-wakeup function being characterised by having

- a sleep phase (power saving mode) correlating with a lower sampling rate, the sleep phase being active when the portable medical device is switched off,

- a wake phase correlating with a higher sampling rate, the wake phase being active when the device is switched on,

- a sleep-to-wake transition, if an impact detected by at least one channel of the impact sensor unit is above a predetermined wakeup threshold level, the sleep-to-wake transition working independent of the switching status of the device.

A typical sampling rate for each channel of the linear acceleration sensor during the wake phase is between 500 Hz and 2000 Hz. A good compromise between energy consumption and reliable detection of impacts occurring during the sleep phase is to reduce the sampling rate of the sensors to a value between 1 Hz and 50, in particular 10 Hz. It was also found that an advantageous wakeup threshold level for linear acceleration is an acceleration level in the range of 3 g to 50 g (detected by at least one channel of the impact sensor unit).

Impact sensor raw data (or any information or results that a classification algorithm running on the controller deduces from the impact sensor unit data) can be stored on an internal electronic memory. Alternatively or in addition it is possible to display an alarm message to the user, if an impact detected by the impact sensor is above a first predetermined alarm threshold level. It is also possible to lock the device in order to prevent a potentially defective device from being used for therapy or diagnosis. Preferably the lock can only be removed by a service technician. Locking the device prevents a patient from starting therapy or monitoring a physical condition using a device, which is prone to malfunction as a consequence of an external crash impact, which has acted on the device previously.

According to another embodiment, it is suggested that the portable medical device comprises an interface that allows for transfer of the impact sensor related data to an external computer. Such an interface is usually already present in up-to-date devices, where it is required to update the software, receive error messages collected during therapy and so forth. According to an even more advanced version of the device, the portable medical device further comprises an additional remote interface, in particular an interface for remote data transfer, for transmitting impact sensor related data to a remote device. This feature can be implemented to improve ease of maintenance of the device by the manufacturer. The remote data transfer requires an interface for data transfer (for example) via internet. Standard data transfer technology can be used to copy the impact sensor related data from the device to a remote server. The manufacturer can use the impact sensor related data that he receives via the remote interface for improved service.

The invention disclosed herein can advantageously improve reliability of any kind of electric portable medical devices. Preferred examples for a portable medical devices according to the invention include a negative pressure wound therapy device, an ambulatory infusion device, in particular an insulin pump, an ambulatory blood pressure unit, an ambulatory pulse monitor, an ambulatory electrocardiogram device, an ambulatory electroencephalogram device and an ambulatory diagnosis system for determining metabolites circulating in the blood.

The present invention includes a method of recognising and/or discriminating impact events, which are detrimental for a portable medical device, comprising the steps

- providing a portable medical device as described in any of the embodiments above,
- detecting an acceleration and/or rotation impact by means of the impact sensor unit,
- executing a classification algorithm, which allows to discriminate a first impact sensor data set, said first impact data set being correlated to a first impact event which is detrimental for the device, from a second impact sensor data set, said second impact data set being correlated to a second impact event which is not detrimental for the device. Said first impact event is a member of the first class of impacts, whereas said second impact event is a member of the second class of impacts.
- recording the result obtained from the classification algorithm,
- optionally displaying an alarm message to the user, if the result obtained from executing the classification algorithm indicates, that an impact event, which is detrimental for the device, has occurred.

The present disclosure also includes a method for creating a hyperplane, such as a separation line, which allows to discriminate sensor data sets, which are correlated to an impact event, which is detrimental for the device from sensor data sets, which are correlated to an impact event, which is not detrimental for the device, comprising the following steps:

   i) Providing a portable medical device as described in any of the embodiments above.
   ii) Experimentally exposing the portable medical device to a series of impact experiments, wherein each impact experiment comprises the step

- recording the linear acceleration values ($a_x$; $a_y$; $a_z$) along the three space axis (x; y; z) and the angular rate values ($\omega_x$; $\omega_y$; $\omega_z$) around the three space axis (x; y; z) during each impact experiment,
- calculating the magnitude $M_\alpha$ and the magnitude $M_\omega$ from the impact sensor data set obtained during each impact experiment,
- entering the magnitude $M_\alpha$ and the magnitude $M_\omega$ into a two-dimensional diagram, thereby creating a data point specific for each impact experiment,
- analysing the technical condition of the portable medical device after each impact experiment,
- classifying each impact experiment as detrimental for the device or as not detrimental for the device, based on the analysis of the technical condition,
- annotating each data point to the classification detrimental or not detrimental.

iii) Creating a separation line (hyperplane) in the two-dimensional diagram, which separates the data points corresponding to a detrimental impact from the data points which are corresponding to a not detrimental impact, whereby a maximum-margin hyperplane is chosen.

It is particularly preferred to use the present invention to improve the reliability of a portable (ambulatory) negative pressure wound therapy device. Accordingly, the inventors are seeking protection for a portable negative pressure wound therapy device adapted to be carried by a user, comprising

- an electrically actuated suction pump for draining wound fluids from a patient,
- at least one mobile electric power supply,
- at least one microelectronic controller for controlling the activity of the electrically actuated suction pump,
- at least one electronic memory,
- at least one multi-channel impact sensor unit, wherein the impact sensor is adapted to detect linear acceleration ($a_x$; $a_y$; $a_z$) along the three space axes (x; y; z) and angular rates ($\omega_x$; $\omega_y$; $\omega_z$) around the three space axes (x; y; z), wherein the impact sensor unit generates impact sensor data sets, and wherein the data generated by the sensor are electronically transferred to the microelectronic controller via an interface,
- a housing, in which the electric components of the device are contained,

characterised in that
the microelectronic controller is adapted to execute a classification algorithm which allows to discriminate a first impact sensor data set, said first impact data set being correlated to a first impact event which is detrimental for the device, from a second impact sensor data set, said second impact data set being correlated to a second impact event which is not detrimental for the device. Said first impact event is a member of the first class of impacts and said second impact event is a member of the second class of impacts.

A method of recognising impact events, which are detrimental for a portable negative pressure wound therapy device adapted to be carried by a user, is also particularly preferred. The method comprises the steps:

i) Providing a portable negative pressure wound therapy device, said device comprising

- an electrically actuated suction pump for draining wound fluids from a patient,
- at least one mobile electric power supply,
- a control unit having at least one microelectronic controller and at least one electronic memory,
- at least one multi-channel impact sensor unit, wherein the impact sensor unit is adapted to detect linear acceleration ($a_x$; $a_y$; $a_z$) along the three space axes (x; y; z) and angular rates ($\omega_x$; $\omega_y$; $\omega_z$) around the three space axes (x; y; z), wherein the impact sensor unit generates impact sensor data sets, and wherein the data generated by the sensor unit are electronically transferred to the microelectronic controller via an interface,
- a housing, in which the electric components of the device are contained,

ii) Detecting an impact by means of the impact sensor,
iii) Executing a classification algorithm, which allows to discriminate a first impact sensor data set, said first impact data set being correlated to a first impact event which is detrimental for the device, from a second impact sensor data set, said second impact data set being correlated to a second impact event which is not detrimental for the device. Said first impact event is a member of the first class of impacts and said second impact event is a member of the second class of impacts.
iv) Recording the result obtained from the classification algorithm, and
v) Optionally displaying an alarm message to the user, if the result obtained from executing the classification algorithm indicates, that an impact event, which is detrimental for the device, has occurred.

A preferred embodiment of present disclosure is also directed to a method for creating a separation line (hyperplane), which allows to discriminate sensor data sets, which are correlated to an impact event, which is detrimental for the negative pressure wound therapy device from sensor data sets, which are correlated to an impact event, which is not detrimental for the negative pressure wound therapy device, comprising the steps of

i) Providing a negative pressure wound therapy device, said negative pressure wound therapy device comprising

- an electrically actuated suction pump for draining wound fluids from a patient,
- at least one mobile electric power supply,
- a control unit having at least one microelectronic controller and at least one electronic memory,
- at least one multi-channel impact sensor unit, wherein the impact sensor unit is adapted to detect linear acceleration ($a_x$; $a_y$; $a_z$) along the three space axes (x; y; z) and angular rates ($\omega_x$; $\omega_y$; $\omega_z$) around the three space axes (x; y; z), wherein the impact sensor unit generates impact sensor data sets, and wherein the data generated by the sensor are electronically transferred to the controller via an interface,
- a housing, in which the electric components of the device are contained,

ii) Experimentally exposing the portable medical device to a series of impact experiments, wherein each impact experiment comprises the step

- recording the linear acceleration values ($a_x$; $a_y$; $a_z$) along the three space axis (x; y; z) and the angular rates ($\omega_x$; $\omega_y$; $\omega_z$) around the three space axis (x; y; z) during each impact experiment,
- calculating the magnitude $M_\alpha$ and the magnitude $M_\omega$ from the impact sensor data set obtained during each impact experiment,
- entering the magnitude $M_\alpha$ and the magnitude $M_\omega$ into a two-dimensional diagram, thereby creating a data point specific for each impact experiment,
- analysing the technical condition of the portable medical device after each impact experiment,
- classifying each impact experiment as detrimental for the device or as not detrimental for the device, based on the analysis of the technical condition,
- annotating each data point to the classification detrimental or not detrimental,

iii) Creating a separation line (hyperplane) in the two-dimensional diagram, said separation line separating the data points into a first class and into a second class of data points, whereby a maximum-margin hyperplane is chosen. Said first class of data points corresponds to a detrimental impact. Said second class of data points corresponds to a not detrimental impact.

Most preferably, the method is performed using a support vector machine.

Execution of classification algorithm on data obtained from impact experiments, to which portable medical devices were subjected, revealed that it is also possible to detect the type of movement or action, to which the portable device was subjected to. Accordingly, if the device is carried by the user during therapy, it is possible for the microcontroller of the device to deduce the type of user activity or operation that the user is performing during that therapy (by executing the classification algorithm). It is, for example, possible for the microcontroller of the device to deduce, by applying the classification algorithm, that the user is driving in a car or that the user is walking. Detection and classification of the type of user activity during a therapy can be used, for example, to improve safety of the therapy for the patient. A user could, for instance, be warned by the device, if he or she enters a condition/activity (such as for example driving in a car, or walking for an extended period of time), that he or she should avoid during the therapy with the portable medical device. A plurality of additional applications, where it is useful to classify the activity (or operation) of a user during a therapy with a portable medical device are conceivable.

Therefore, the present disclosure is also directed to a classification of a user activity during a medical therapy. The method comprises the steps:

- providing a portable medical device as described in any of the embodiments herein,
- recording the linear acceleration values ($a_x$; $a_y$; $a_z$) along the three space axis (x; y; z) and the angular rate values ($\omega_x$; $\omega_y$; $\omega_z$) around the three space axis (x; y; z) during the therapy,
- preferably calculating the magnitude $M_\alpha$ and the magnitude $M_\omega$ from the impact sensor data set obtained during each impact,
- executing a classification algorithm, in particular a classification algorithm as explained herein, whereby the classification algorithm is used to detect the type of user activity during the therapy
- optionally displaying an alarm message to the user, if the result obtained from executing the classification algorithm indicates, that a user enters a condition, which is not recommended during the type of therapy applied.

Preferably, classification is applied using a support vector machine algorithm.

BRIEF DESCRIPTION OF THE DRAWINGS

Further characteristics, details, and advantages of the invention result from the appended patent claims and from the drawings and the following description of preferred embodiments of the invention. The drawings show a negative pressure wound therapy device as preferred (but non-limiting) example of a portable medical device. The invention can be used for many other kinds of portable medical devices as described in detail in the above chapters of the description.

The drawings show:

FIG 1 A schematic drawing of a simple negative pressure wound therapy device including the negative pressure bandage applied to a wound of a patient.

FIGS. 2 a to e Different views of a typical portable negative pressure wound therapy device to generate a vacuum for medical applications.

FIG 3 A schematic drawing of the piping system and of the electronic components of a negative pressure wound therapy device according to a preferred embodiment of the present invention.

FIG 4 A schematic drawing of the structure of the electronic control system and of the electric/electronic/eletromechanical components of a portable negative pressure wound therapy device according to a preferred embodiment of the invention.

FIG 5 A schematic drawing of the structure of a stand-alone impact sensor unit module according to a preferred embodiment of the invention.

FIG 6 Example of a warning message displayed to the user of a portable negative pressure wound therapy device according to a preferred embodiment of the invention.

FIGS. 7 a - f Signal magnitudes determined from impact experiments ("training experiments"). The charts show the values of the magnitudes $M_\alpha$ and $M_\omega$ in relative units.

7 a Distribution of signal magnitudes from 102 impact experiments

7 b Subset "pendulum experiment" (pool of EXP 1 A/B)

7 c Subset "tilting experiment" (EXP 8)

7 d Subset "stairs experiment" (pool of EXP 9 A/B)

7 e Subset "chair experiment" (EXP 7)

7 f Subset "driving experiment" (EXP 8)

FIGS. 8a, b Acceleration forces and angular rates detected during a single impact event. From the detected linear acceleration forces along the three axis the magnitude $M_\alpha$ was calculated (FIG 8 a). Rotation magnitude $M_\omega$ was calculated (FIG 8 b) from the gyration sensor recordings made during the same drop experiment.

DESCRIPTION OF THE PREFERRED EMBODIMENT

Preferred embodiments of the invention are described by means of a negative pressure wound therapy device. A mobile negative pressure wound therapy device serves as a non-limiting example for a portable medical device of the invention.

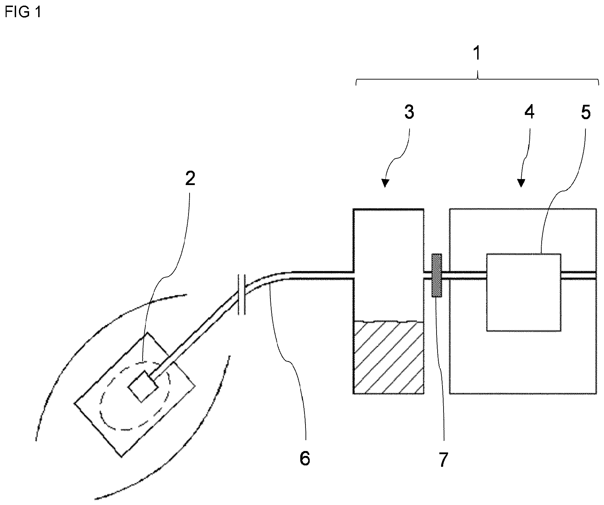

A schematic representation of a simple negative pressure wound therapy device 1 is shown in FIG 1. The therapy device is in fluid communication with a wound 2 of a patient to be treated. The device 1 comprises a container 3 for collecting the fluids (such as blood, instillation fluids and the like) that are sucked from the wound 2. Wound therapy devices of this type are known in the prior art. The container (or canister) 3 is typically made of a solid material, such as a plastic material. It is usually a disposable article designed for single use. Conveniently, the container 3 can be detachably mounted to the housing part 4 of the device, which contains the electrical components of the apparatus. The container 3 can be evacuated by the electrically actuated suction pump 5. A connection (not shown) is provided for a suction line 6 that leads to the wound such that vacuum communication can be established between the suction pump 5, the container 3, and the suction line 6 that leads to the wound. A filter or air/liquid-separator 7 located within the fluid-pathway between the container 3 and the suction pump 5 is used to prevent exudate from being sucked into the pump 5. A negative pressure wound therapy device typically comprises additional components such as a control system for controlling activity of the pump and means for interacting with the user, such as a touch-screen display or control buttons. These components are not shown in FIG 1.

In some embodiments, the portable negative pressure wound therapy device does not have a container for receiving the drained body fluids. Instead, the body fluids can be contained, for example, in the dressing. This is achieved by providing absorbent layers (not shown in FIG 1). Such negative pressure wound therapy devices, which do not make use of a separate solid exudate canister are typically used for treating less exudating wounds, for example surgical wounds.

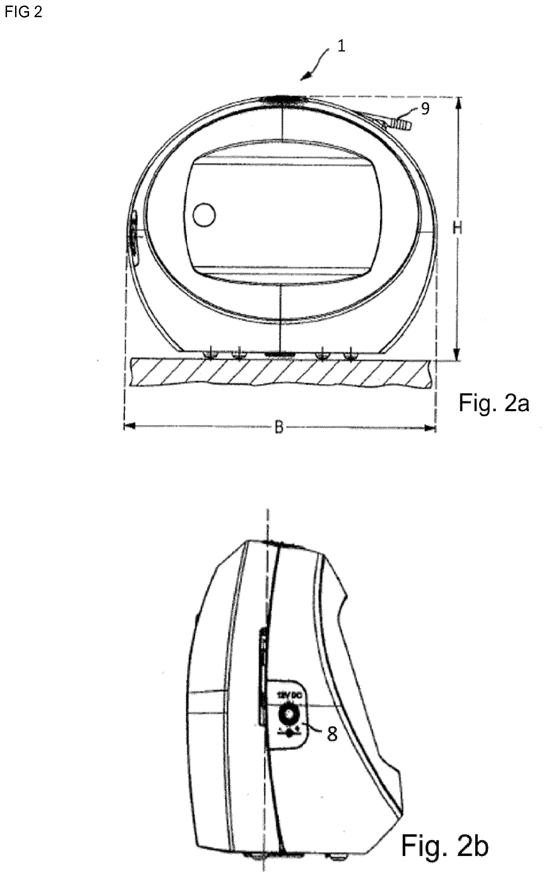

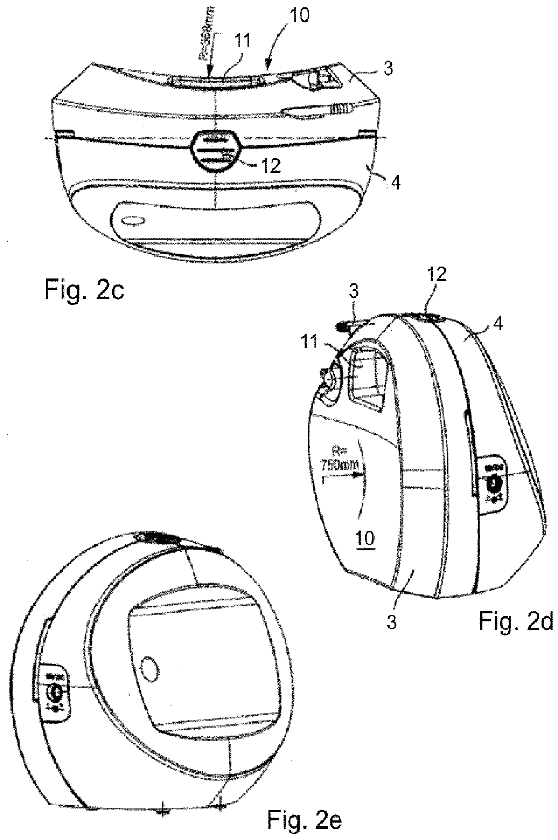

FIGS. 2 a to e show a typical example of a portable device 1 for the provision of the vacuum for medical applications. The device 1 comprises a first housing part 4 in which a vacuum-producing device in the form of an air suction pump as well as the electrical components for the device are accommodated, including batteries or preferably rechargeable batteries. A recharging connection for the batteries is designated by reference symbol 8. Moreover, the device 1 comprises a second housing part in the form of a container 3 for receiving body fluids, in particular, for receiving wound exudates suctioned away from a wound. Preferably, the entire second housing part is constituted as a disposable single-use item. In its upper region, a connection gland 9 for a suction tube is provided that may, for example, lead to a wound dressing sealing the wound when the device 1 is used in the vacuum therapy of wounds and there it can, for example, communicate with the wound space through a port to apply and maintain a vacuum to the wound space and to suction away wound exudates into the container. For this purpose, the container 3 communicates with the suction pump 5.

It can also be seen from FIG 2d on the side 10 of the second housing part 3 facing the body, a grip recess 11 is formed in the shape of an opening extending right through the second housing part 3. In this way, the device 1, or only its second housing part 3, can be gripped and handled with one hand.

In the preferred embodiment shown, a manually operable element 12 is provided near this grip recess 11 on the upper side of the device 1, for example, in the form of a pushbutton that acts on locking and back-gripping means (not shown). In the joined condition of the two housing parts 3 and 4, the locking or back-gripping means are in a locked condition holding the two housing parts 3, 4 together by positive action. Only upon operation of the operating element 12, the lock is released so that the housing parts 3, 4 can be separated from each other.

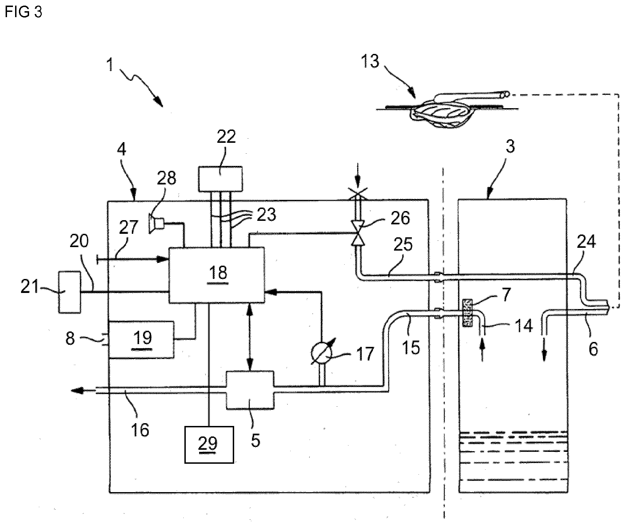

FIG. 3 shows the nature of the piping system and of the electric components (including electronic and/or electromechanical components) of a negative pressure wound therapy device according to a preferred embodiment of the invention. According to this preferred embodiment, the device comprises an inventive microelectronic impact sensor, wherein the impact sensor is adapted to detect an acceleration impact acting on the device. Apart from the inventive feature, the device is similar to negative pressure wound therapy device of the type exemplified in FIG 2. In contrast to the very basic system shown in FIG 1, the device of FIG 3 includes additional components (known from the art) such as the air rinsing pathway of the fluid system. Based on FIG 3 one embodiment of the inventive device is explained. FIG 3 shows the previously described or a similar device for providing a vacuum for medical applications in a purely schematic representation, wherein relevant reference symbols are used for the corresponding components. However, FIG 3 shows only the components that are relevant for the following description. FIG 3 depicts a schematically indicated wound to be treated with a vacuum with a vacuum-tight wound dressing 13, to which the suction tube 6 emanating from the container 3 leads. From the container 3, a further tube section 14 leads outwardly through the already mentioned filter 7. If the container 3 or the first housing part 4 is put into its operating position on the first or basic housing part 4 of the device 1, the tube section 14 is connected to a further tube section 15 within the first housing part 4 that leads to the intake side of the suction pump 5. When the suction pump 5 operates, a vacuum is applied to the container 3 and to the suction tube 6 via tube sections 14, 15.

Moreover, a pressure sensor 17 for measuring the pressure is provided in the tube section 15 between container 3 and suction pump 5. Its signals are sent to an electronic control unit, collectively identified by reference symbol 18, which performs open-loop and closed-loop control of the device 1 in total. The electronic control unit 18 comprises a microelectronic controller and at least one electronic memory. Also shown is the charging connection 8 for rechargeable batteries that are located in a compartment 19 and a connection 20 for a schematically indicated power supply unit 21. Reference symbol 22 indicates a display unit, preferably having a capacitive switch membrane (touchscreen). A user may control operation of the device via said touchscreen. The electrical connection to the electronic control unit 18 is indicated via electrical lines 23. The suction pump 5 is controlled by the electronic control unit 18 in which, by means of the signals of the pressure sensor 17, a pressure and vacuum closed-loop control is implemented with known open-loop and closed-loop control mechanisms (set point/actual value control mechanisms), so that the pressure value corresponding to the currently selected program is controlled in the tube section 15.

Also shown is an additional rinsing or aeration tube 24 that, only shown by way of example, leads through the container 3 and just like the suction tube 6 leads to the wound dressing 13. When the container 3 is attached in its intended assembly position on the first housing part 4, this rinsing tube 24 communicates with a tube section 25 provided in the first housing part 4 in which an electromagnetically operated valve 26 is provided that can be actuated by the electronic control unit 18 and connects the tube section 25 with the atmospheric air when it is open, so that an air current toward the wound via the rinsing tube 24 can be generated.

The device 1 and its electronic control unit 18 also feature a data interface 27, preferably a USB interface, by means of which the electronic control unit 18 or its method of operation can be programmed. In addition, device 1 comprises a speaker 28 which is connected to the control unit 18. The speaker can be used to generate acoustic alarm signals.

According to the invention, the portable negative pressure wound therapy device further comprises a multi-channel impact sensor unit 29, which is capable of communicating with electronic control unit 18. As explained above, the electronic control unit 18 comprises a microcontroller. The multi-channel impact sensor unit 29 has three linear acceleration sensor channels as well as gyration sensors having three channels for angular rotation. The IMU motion sensor LSM9DS0 from ST Microelectronics, United Kingdom, can be used. Advantageously, the LSM9DS0 additionally comprises a magnetic field sensor, which can be used to detect magnetic fields that could harm the electronic components of the device.

In the example shown in FIG 3, the multi-channel impact sensor unit 29 communicates directly, by means of an interface (not shown), with the electronic control unit 18. In this embodiment (herein called an "integrated impact sensor"), the impact sensor unit 29 is an integrated component of the control system 18 of the device. Also, the impact sensor data are processed by the electronic control unit 18 and the sensor raw data and/or the processed data storage is controlled by the electronic control unit 18. The integrated impact sensor unit 29 is usually (but not necessarily) provided with electric energy by the main power supply system of the device.

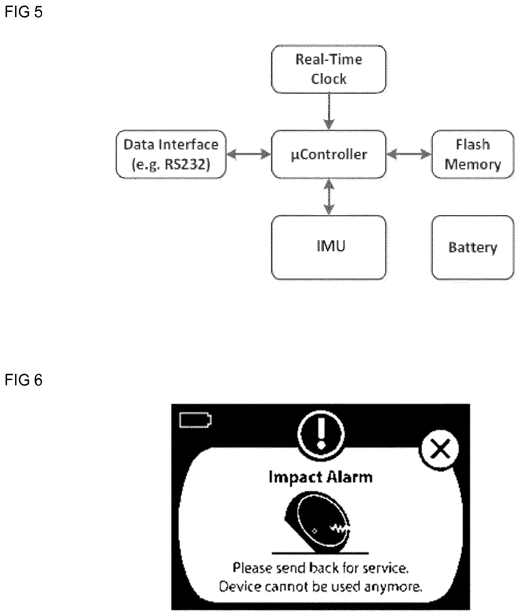

More preferably (unlike the example shown in FIG 3), the multi-channel impact sensor unit is a component of a stand-alone impact sensor unit module. A stand-alone impact sensor unit module is explained in more detail in FIG 5 below.

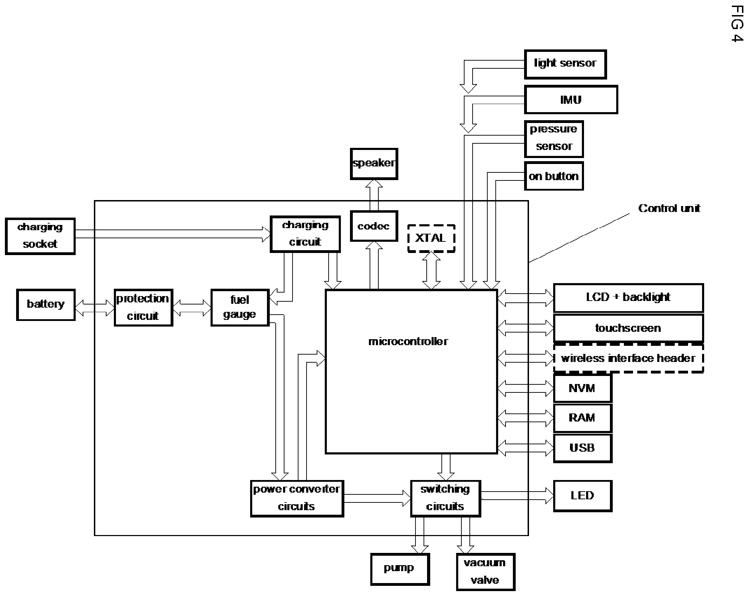

Interaction of the multi-channel impact sensor unit 29 with the control unit 18 is also shown in FIG 4 below.

Multi-channel impact sensor unit 29 can conveniently be mounted to the same main board, on which electronic control unit 18 is sitting.

Proceeding to FIG 4, communication of the control unit with peripheral components is shown schematically. One peripheral component communicating with the controller is the integrated multi-channel impact sensor unit (termed "IMU" in FIG 4). Instead of an integrated multi-channel impact sensor unit a stand-alone impact sensor module can be used likewise.

The control unit includes elements/functions such as the microelectronic controller ("microcontroller"), a protection circuit for the power supply, a fuel gauge for the power supply, a charging circuit for the power supply, power converter circuits, a speaker controller and switching circuits for controlling the suction pump and the electromagnetic valve.

The peripheral electric/electronic components include a source of electric energy, preferably a rechargeable battery, the suction pump, preferably a membrane pump and at least one electronic memory typically including both ROM and RAM chips. In up-to date devices, the peripheral electric/electronic components may additionally include, for example, a vacuum valve for a rinsing fluid pathway (as exemplified in FIG 3), interfaces such as a USB connector, electric connectors, such as a connector for the battery charger, a light sensor, a pressure sensor for sensing the pressure within the closed fluid-system of the device, a speaker for signalling warnings to the user, one or more LEDs for signalling warnings and/or status information to the user, a display, preferably a touchscreen display for interacting with the user, a backlight for the display, buttons, such as an on/off button. More advanced peripheral components such as a wireless interface for communication of the control unit with a remote computer may optionally be present (box with dotted line in FIG 4).

The control unit is the central instance for controlling and monitoring function of the device.

According to the invention the peripheral components include a multi-channel impact sensor unit (IMU), which may be an integrated impact sensor unit or preferably a stand-alone impact sensor module, each of which communicate with the microcontroller via an interface. The microcontroller receives impact sensor data (i.e. sensor data from any of the six channels of the multi-channel impact sensor unit). If an integrated impact sensor unit is used, the microcontroller receives the sensor raw data from the 6 channels. The raw data are processed as explained in detail above. If a stand-alone impact sensor module is used, the microcontroller receives the processed data including the classification results and may, for example, display warnings to the user or lock the device.

The stand-alone impact sensor module shown in FIG 5 comprises at least one multi-channel impact sensor unit (IMU) and further a microelectronic controller (in the following called "impact sensor module controller"; in FIG 5 the impact sensor module controller is shortly termed "μController") cooperating with the IMU. The impact sensor module controller functions independent of the microelectronic controller of the control unit. The module further comprises a data interface, such as a RS232 interface, a memory, such as a flash memory and a real-time clock. The sensor, the interface, the memory and the real-time clock are in direct electronic communication with the impact sensor module controller. A separate battery or accumulator supplies electric energy to the components of the module thus making the module independent of the charging status of the main battery of the device. A particular advantage of using a stand-alone impact sensor module is, that the module can function independently of the working status of the device. The stand-alone impact sensor module can be active even if the device is switched off, as described in connection with the preferred embodiments herein. Thus, the stand-alone impact sensor module may perpetually report detrimental impacts that were acting on the device, for example also during storage of the device or during transport of the device.

FIG 6 shows an example of a warning message that is signalled to the user by means of a LCD-display. Such a message can be displayed after the controller of the device has classified the data received from the impact sensor unit as "detrimental for the device" (first class). The alarm message according to this example informs the user that an impact was acting on the device and furthermore that the device is locked. Unlocking of the device can only be accomplished by a service technician authorised by the supplier/manufacturer. Before unlocking the device the service technician will perform a careful inspection of the device, optionally in combination with a prophylactically exchange of those components, which are typically prone to deficiency after a detrimental impact. Hereby the service technician can also utilise information about the strength of the impact, which can be stored in the memory of the device.

EXAMPLE 1

In order to establish an exemplary training data set for a portable medical device (here: a mobile negative pressure wound therapy apparatus), the signal magnitudes (linear acceleration as well as rotation) were determined from a plurality of impact experiments (in total 102 experiments). Signal magnitudes are given as relative values. During the experiments the device (portable negative pressure wound therapy unit "VivanoTec" ®) was subjected to impacts, which are usually detrimental for the device (first class of impacts) as well as to impacts, which are usually not detrimental for the device (second class of impacts).

The medical device was subjected to the following types of impacts. The number of experiments is shown in brackets:

EXP 1A Horizontal crash of display against a wall, while device is swinging from its carrying belt. This kind of crash occurs very often, if a user carries the device around his/her neck (5).

EXP 1B Horizontal crash of backside/container against a wall, while device is swinging from its carrying belt. Same as 1A this kind of crash occurs very often, if a user carries the device around his/her neck (4).

EXP 2A Crash to carpet from height of 100 cm with empty exsudate container (10).

EXP 2B Crash to steel plate from height of 100 cm with empty exsudate container (3).

EXP 3 Crash to steel plate from height of 100 cm with full 800 ml exsudate container (10).

EXP 4 Same as EXP 2B (10).

EXP 5 Crash to steel plate from height of 50 cm with empty exsudate container (10).

EXP 6 Crash to steel plate from height of 25 cm with empty exsudate container (10).

EXP 7 Patient sitting down to a chair (10).

EXP 8 Device was overturned (90° turn) while standing on a table (10).

EXP 9A Patient walking/jumping down a stair in a fast mode (5).

EXP 9B Patient walking down a stair in a slow mode (5).

EXP 9B Patient was driving in a car including driving on a dirt road (10).

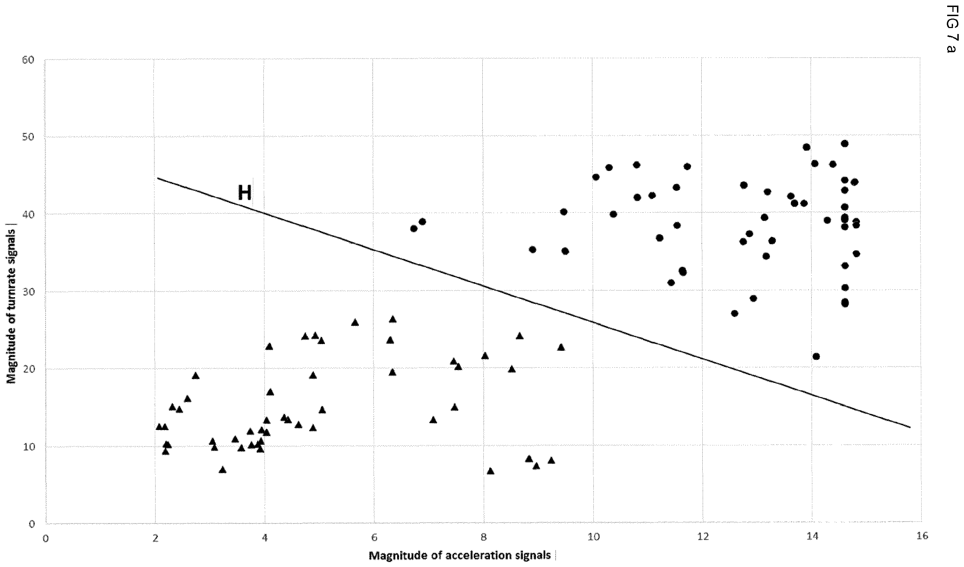

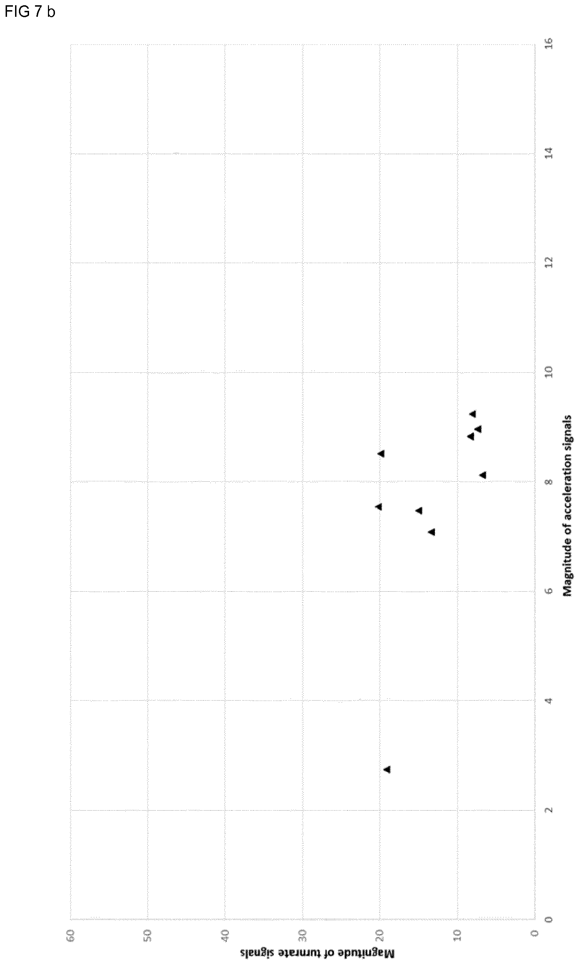

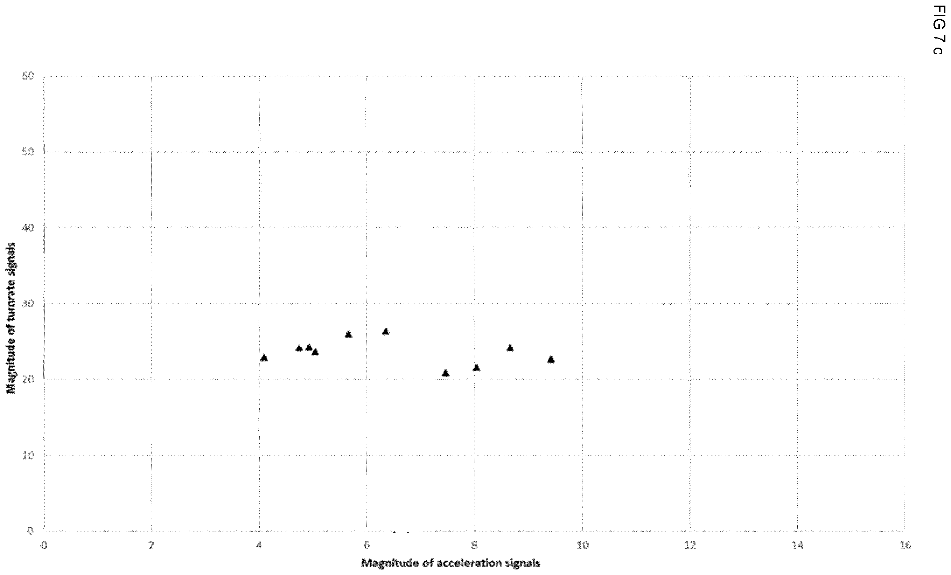

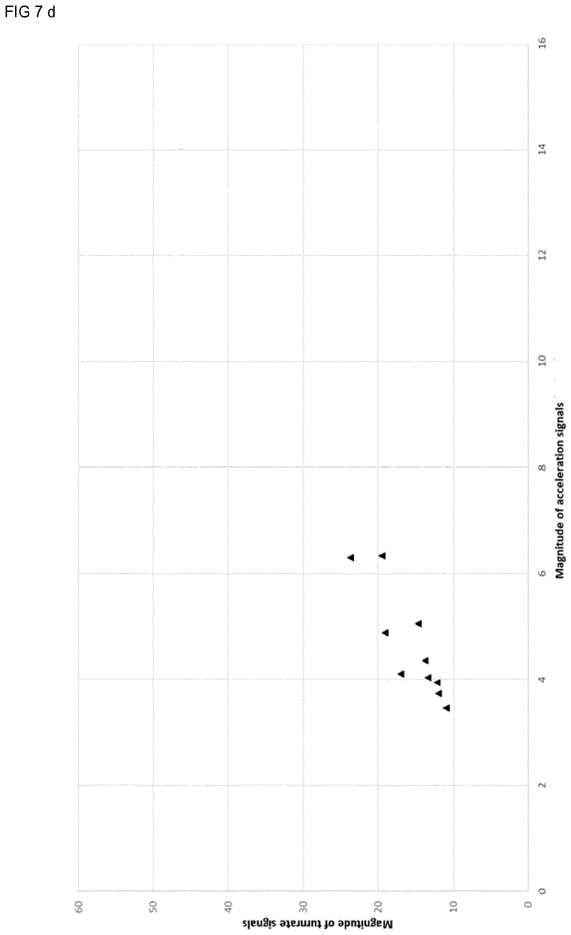

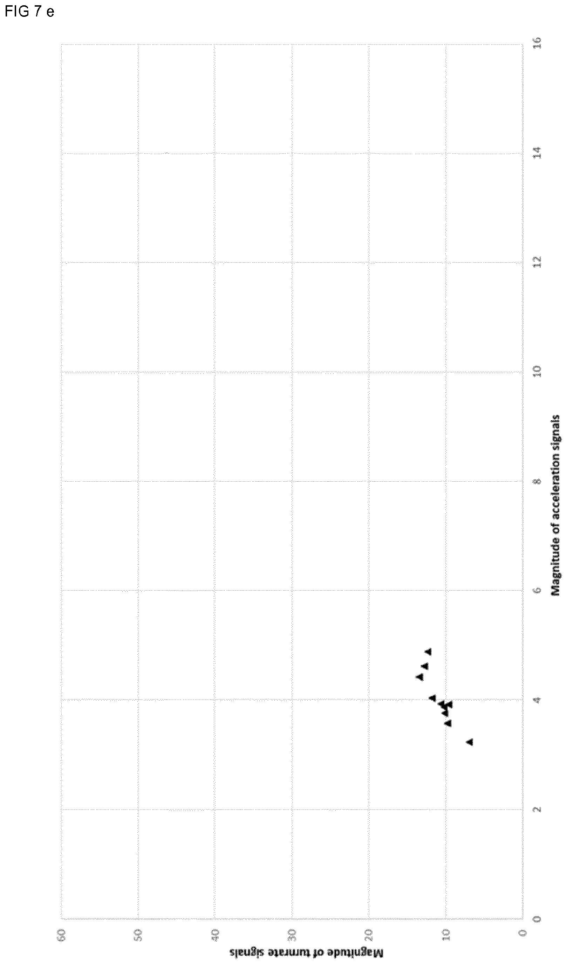

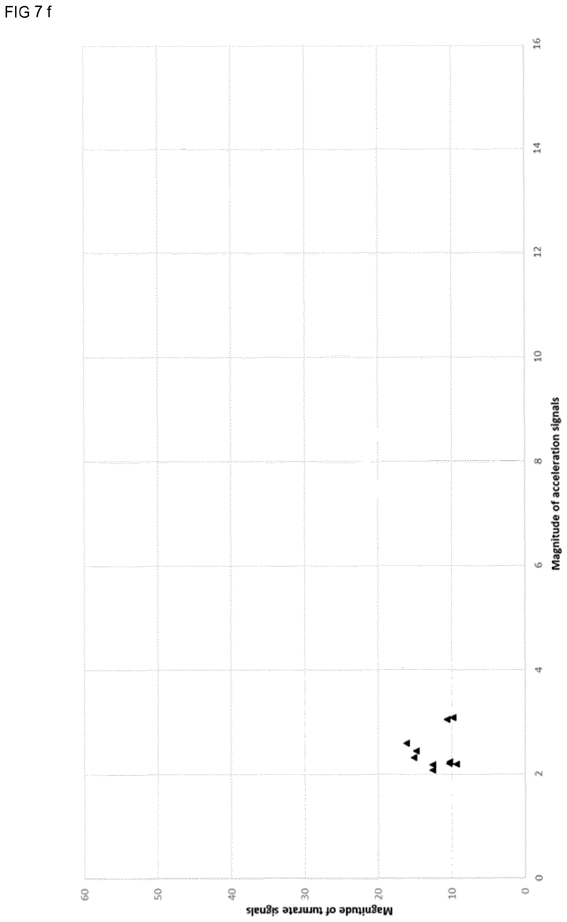

FIG 7 a shows the distribution of signal magnitudes from 102 impact experiments, of which 53 (round dots) belong to the first class of impacts and 49 (triangles) belong to the second class of impacts.

Sensor data were recorded by means of an inertial sensor module mounted to the main board of a portable negative pressure wound therapy device (VivanoTec® negative pressure unit from Paul Hartmann AG, Germany). For the experiments the IMU motion sensor LSM9DS0 (iNEMO inertial module) from ST Microelectronics, United Kingdom was used. This inertial module device has three acceleration channels, three angular rate channels and three magnetic field channels. Data from the magnetic field channels were not recorded. From the detected acceleration forces and angular rates the magnitudes $M_\alpha$ and $M_\omega$ were calculated. The chart given in FIG 7a shows each impact event mapped according to its maximum values of the magnitudes $M_\alpha$ (x-axis) and $M_\omega$. (y-axis) observed during the impacts.

Experiments 2A, 2B, 3, 4, 5, 6 belong to the first class of impacts, which are usually detrimental for the device ("dropping to a ground" type of impacts). The events resulting from experiments 2A, 2B, 3, 4, 5, 6 are represented by the round dots. The other experiments 1A, 1B, 7, 8, 9, 10 shown belong to the second class of impacts, which are usually not detrimental for the device. During such kinds of impacts, which occur regularly, when the portable device is used outside of a medical facility, may also be accompanied by significant acceleration and rotation movements. However, a portable medical device is usually constructed to withstand such impacts without being harmed. The events resulting from experiments 1A, 1B, 7, 8, 9, 10 are represented by the triangles.

The events corresponding to the first class (round dots) form a clearly recognisable group. Likewise, which the events corresponding to the second class (triangles) form another coherent group. The first and the second classes do not overlap. It is possible to separate the classes by a linear classifier, namely hyperplane "H" shown in FIG 7h. Hyperplane H should is chosen with maximum margins to both classes of events. Preferably a support vector machine algorithm is used to construct the hyperplane. Any new impact event detected by the impact sensor system of the portable device can be mapped into the diagram and will fall to either side of the hyperplane resulting in a classification "detrimental" or "not detrimental".

The experiments show that the first and the second class of impacts are clearly discriminable by a simple classification approach based on plotting the magnitudes $M_\alpha$ and $M_\omega$ (as explained more in detail above) in a two-dimensional plane. Hyperplane (marked as "H" in FIG7a) can be generated using a standard support vector machine approach.

If a linear separation of the two classes should not be possible (which was not observed in the experiments done by the inventors) known Kernel methods can be used to establish a separator.

In order to collect training data sets, it is necessary to repeatedly perform experiments of the type described in this example. Training experiments will have to be done for each type of device, because classification depends on device specific parameters like stability, weight, material and shape of the device. Reliability of classification increases with the number of training experiments.

The experiments also demonstrate the usefulness of the classification approach based on a plurality of sensor signals (i.e. from the six sensor channels) in combination with calculating the acceleration and rotation vectors (i.e. magnitudes $M_\alpha$ and $M_\omega$). As can be seen from diagram FIG 7a a discrimination of the first and the second class of impacts would not be possible, if only linear acceleration sensor date would be used: The acceleration magnitudes $M_\alpha$ corresponding to experiments EXP 1A, 1B, 8 (see FIG 7b and 7c, which belong to the first class, i.e. not detrimental) overlap with the magnitudes $M_\alpha$ corresponding to experiments EXP 2A, 2B, 3, 4, 5, 6 (belonging to the first class, i.e. detrimental). Only the second dimension, i.e. the rotation vectors, allows for a linear discrimination. Similarly, it would not be possible to find a linear separator dividing the first group from the second class by using the rotation vectors alone.

Figures 7b - f show subsets of the results for the different types of not detrimental impacts. Data sets of the "pendulum experiments" EXP 1 A/B (FIG 7b) as well as of the "stairs experiments" EXP 9 A/B (FIG 7d) were pooled, respectively. FIG 7c shows the data sets from the tilting experiment EXP 8, where an apparatus was overthrown. This group of EXP 8 is characterised by relatively similar rotational magnitudes $M_\omega$, whereas the acceleration magnitudes $M_\alpha$ exhibit a comparatively stronger variability. The subset shown in FIG 7e represents the "chair experiment" EXP 7. Data from subset "driving experiment" EXP 8 can be seen in FIG 7f.

All different data subsets form clearly discernible groups, when the magnitude of the rotation rates $M_\omega$ is plotter against the magnitude of the linear acceleration magnitudes $M_\alpha$. The experiments shown herein demonstrate, that a classification algorithm can be used to assign impact sensor data sets to specific user actions. Classification and detection of user action could be used, for example, to warn a user if he enters a condition, which is not recommended during the type of therapy applied.

EXAMPLE 2

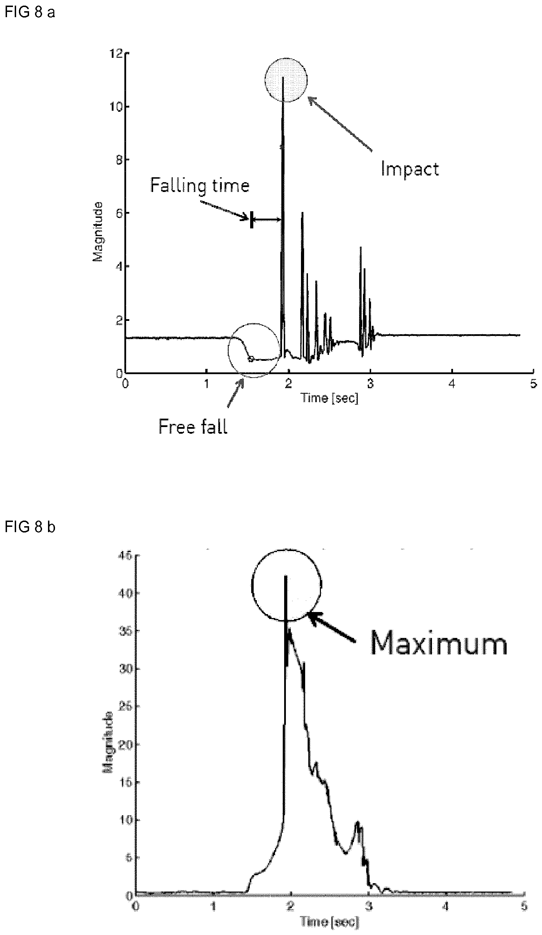

FIGs 8a, b shows an example of processed impact sensor data recorded by means of an inertial sensor module mounted to the main board of a portable negative pressure wound therapy device (VivanoTec® negative pressure unit from Paul Hartmann AG, Germany). For the experiments the same IMU motion sensor as the one used for the experiments described in example 1 was used (LSM9DS0 inertial module from ST Microelectronics). As in example 1, data from the magnetic field channels were not recorded.

The data corresponding to FIG 8a, b were received while the device was subjected to a drop test. During the test the device was dropped to a soft ground (rubber plate) from a height of 1,0 m above ground. A maximum magnitude $M_\alpha$ value > 11 (relative units) was observed. A drop of this kind in many cases does not leave any damages visible from the outside to the device. However, the acceleration occurring during the drop imposes stress to internal mechanical or electronic components, such as electric contacts or tube connections, which may lead to a malfunction during future therapy cycles. Accordingly, a drop of this category has to be classified as an "impact event, which is detrimental for the device".

The data corresponding to FIG 8b shows magnitude $M_\omega$ reflecting the overall gyration/rotation forces that the device was exposed to during the same experiment (drop to a soft ground) already described under FIG 8 a. A maximum magnitude $M_\omega$ value of > 42 (relative units) was observed. A comparison of FIG 8 a with FIG 8b shows that the peak of acceleration magnitude $M_\alpha$ coincides with the peak of gyration magnitude $M_\omega$. This indicates that the crash entails both strong linear acceleration and strong rotation forces, which occur simultaneously.

FIG 8 a also shows the incidents occurring during the impact experiment: The free fall phase of the experiment, during which gravity approaches towards zero (therefore minimising magnitude $M_\alpha$, if the device is dropped vertically) is indicated by a first circle. The free fall ends with the first impact to the ground leading to the acceleration peak. After the drop, the height of the fall can be estimated, using the recorded acceleration values and a standard function analysis algorithm. The algorithm is adapted to detect the begin of the free fall (tff; first downward turning point of the acceleration function) and the begin of the impact ($t_{im}$, first upward turning point of the acceleration function). The height of the fall can then be calculated using the formula

$$s \text{ (height)} = g \text{ (gravity)} \times (t_{im} - t_{ff})^2$$

For the estimation of the height of the fall the air resistance is disregarded. In the case shown in FIGs 8 a the time span of the free fall (taken from the diagram) is about 0,33 s leading to an estimated drop height of 1,07 m [9.81 m/s$^2$ x (0,33 s)$^2$ = 1,07 m], which is a good approximation to the actual value of 1.00 m.

**Claims**

1. A portable medical device (1) adapted to be carried by a user, comprising

   - at least one electrically actuated means (5) for performing a therapeutic and/or a diagnostic function on a patient,
   - at least one mobile electric power supply (21),
   - a control unit (18) having at least one microelectronic controller and at least one electronic memory,
   - at least one multi-channel impact sensor unit (29), wherein the impact sensor unit (29) is adapted to simultaneously detect linear acceleration $(a_x; a_y; a_z)$ along the three space axes $(x; y; z)$ and angular rates $(\omega_x; \omega_y; w_z)$ around the three space axes $(x; y; z)$, wherein the impact sensor unit (29) generates impact sensor data sets, and wherein the impact sensor data sets are electronically transferred to the microelectronic controller via an interface,
   - a housing (4), in which the electric components of the device (1) are contained,

   **characterised in that**
   the microelectronic controller is adapted to execute a classification algorithm which allows to discriminate

   - a first impact sensor data set, said first impact data set being correlated to a first impact event which is detrimental for the device (1), from
   - a second impact sensor data set, said second impact data set being correlated to a second impact event which is not detrimental for the device (1),

   wherein the classification algorithm includes a support vector machine using a two-dimensional space and a one-dimensional separation line (hyperplane "H"),
   wherein the first dimension of the two dimensional space is defined by the magnitude $M_\alpha$ corresponding to the vector length of the linear acceleration values $(a_x; a_y; a_z)$ measured by the acceleration sensor and wherein the second dimension of the two dimensional space is defined by the magnitude $M_\omega$ corresponding to the vector length of the angular rate values $(\omega_x; \omega_y; \omega_z)$ measured by the rotation sensor.

2. Portable medical device (1) according to claim 1 any one of the preceding claims, wherein the impact sensor unit (29) comprises a microelectronic inertial measurement unit (IMU) for detection of linear acceleration and rotation and wherein the microelectronic inertial measurement unit (IMU) is integrated in a single chip.

3. Portable medical device (1) according to any one of the preceding claims, wherein the at least one electronic memory is adapted to store operating instructions for the microelectronic controller and/or to record status information related to the function of the device.

4. Portable medical device (1) according to claim 3, wherein the at least one electronic memory is further adapted to store sensor data sets generated by the at least one impact sensor unit (29) and/or to store a result obtained by the classification algorithm.

5. Portable medical device (1) according to any one of the preceding claims, wherein the at least one electronic memory comprises a separate electronic impact sensor data memory, in particular a flash memory, wherein the separate electronic impact sensor data memory is adapted to store impact sensor data sets generated by the at least one impact sensor unit (29) and/or to store a result obtained by the classification algorithm.

6. Portable medical device (1) according to any one of the preceding claims, wherein the device (1) further comprises a separate mobile electric power supply (21), in particular a battery, a rechargeable accumulator or a capacitor, wherein the separate electric power supply (21) is adapted to supply electric energy to the impact sensor unit (29).

7. Portable medical device (1) according to any one of the preceding claims, wherein the impact sensor unit (29) is adapted to activate an inactive microelectronic controller, if the impact sensor unit (29) detects at least one linear acceleration value $(a_x; a_y; a_z)$ or at least one angular rate value $(\omega_x; \omega_y; w_z)$ above a predetermined threshold level.

8. Portable medical device (1) according to any one of the preceding claims, further comprising a real time clock, whereby the device (1) is adapted to record the time of an impact, if the impact sensor unit (29) detects at least one linear acceleration value $(a_x; a_y; a_z)$ or at least one angular rate value $(\omega_x; w_y; w_z)$ above a predetermined threshold level.

9. Portable medical device (1) according to any one of the preceding claims, further comprising a real time clock, whereby the device is adapted to record the time at which the classification algorithm has produced a discrimination result.

10. Portable medical device (1) according to any one of the preceding claims, wherein the microelectronic controller comprises a user interface (27) adapted to receive user settings and/or to display one or more system conditions.

**11.** Portable medical device (1) according to any one of the preceding claims, **characterized in that** the device (1) is selected from the group of an ambulatory wound therapy device, in particular a negative pressure wound therapy device (1), an ambulatory deep brain stimulation device, an ambulatory cardiac stimulator, an ambulatory infusion device, in particular an insulin pump, an ambulatory blood pressure unit, an ambulatory pulse monitor, an ambulatory electrocardiogram device, an ambulatory electroencephalogram device and an ambulatory diagnosis system for determining metabolites circulating in the blood.

**12.** Method of discriminating a first and a second impact event, comprising the steps

- providing a portable medical device (1) according to any one of the preceding claims,
- detecting the first or a second impact event by means of the impact sensor unit (29),
- executing a classification algorithm, which allows to discriminate a first impact sensor data set, said first impact data set being correlated to a first impact event which is detrimental for the device (1) from a second impact sensor data set, said second impact data set being correlated to a second impact event which is not detrimental for the device (1),
- recording the result obtained from the classification algorithm,
- optionally displaying an alarm message to the user, if the result obtained from executing the classification algorithm indicates, that a first impact event, which is detrimental for the device (1), has occurred.

**Patentansprüche**

**1.** Tragbare medizinische Vorrichtung (1), die dazu eingerichtet ist, durch einen Benutzer getragen zu werden, und die Folgendes umfasst:

- wenigstens ein elektrisch betätigtes Mittel (5) zum Durchführen einer therapeutischen und/oder diagnostischen Funktion an einem Patienten,
- wenigstens eine mobile elektrische Leistungsversorgung (21),
- eine Steuereinheit (18) mit wenigstens einer mikroelektronischen Steuerung und wenigstens einem elektronischen Speicher,
- wenigstens eine Mehrkanalaufprallsensoreinheit (29), wobei die Aufprallsensoreinheit (29) dazu eingerichtet ist, gleichzeitig eine Linearbeschleunigung ($a_x$; $a_y$; $a_z$) entlang der drei Raumachsen (x, y; z) und Drehraten ($w_x$; $\omega_y$; $w_z$) um die drei Raumachsen (x, y, z) herum zu detektieren, wobei die Aufprallsensoreinheit (29) Aufprallsensordatensätze erzeugt und wobei die Aufprallsensordatensätze elektronisch über eine Schnittstelle an die mikroelektronische Steuerung übertragen werden,
- ein Gehäuse (4), in dem die elektronischen Komponenten der Vorrichtung (1) enthalten sind,

**dadurch gekennzeichnet, dass**
die mikroelektronische Steuerung dazu eingerichtet ist, einen Klassifizierungsalgorithmus auszuführen, der es ermöglicht, Folgendes zu unterscheiden:

- einen ersten Aufprallsensordatensatz, wobei der erste Aufpralldatensatz mit einem Aufprallereignis korreliert ist, das für die Vorrichtung (1) schädlich ist, von
- einem zweiten Aufprallsensordatensatz, wobei der zweite Aufpralldatensatz mit einem zweiten Aufprallereignis korreliert ist, das für die Vorrichtung (1) nicht schädlich ist,

wobei der Klassifizierungsalgorithmus eine Unterstützungsvektormaschine (Support Vector Machine) beinhaltet, die einen zweidimensionalen Raum und eine eindimensionale Separationslinie (Hyperebene "H") verwendet, wobei die erste Abmessung des zweidimensionalen Raums durch den Betrag $M_a$ definiert ist, der der Vektorlänge der Linearbeschleunigungswerte ($a_x$; $a_y$; $a_z$), die durch den Beschleunigungssensor gemessen werden, entspricht, und wobei die zweite Abmessung des zweidimensionalen Raums durch den Betrag $M_\omega$ definiert ist, der der Vektorlänge der Drehratenwerte ($\omega_x$; $\omega_y$; $\omega_z$), die durch den Rotationssensor gemessen werden, entspricht.

**2.** Tragbare medizinische Vorrichtung (1) nach Anspruch 1 einem der vorhergehenden Ansprüche, wobei die Aufprallsensoreinheit (29) eine mikroelektronische Inertialmesseinheit (IMU: Inertial Measurement Unit) zur Detektion einer Linearbeschleunigung und Rotation umfasst und wobei die mikroelektronische Inertialmesseinheit (IMU) in einem einzigen Chip integriert ist.

**3.** Tragbare medizinische Vorrichtung (1) nach einem der vorhergehenden Ansprüche, wobei der wenigstens eine elektronische Speicher dazu eingerichtet ist, Betriebsbefehle für die mikroelektronische Steuerung zu speichern und/oder Statusinformationen bezüglich der Funktion der Vorrichtung aufzuzeichnen.

**4.** Tragbare medizinische Vorrichtung (1) nach Anspruch 3, wobei der wenigstens eine elektronische Speicher ferner dazu eingerichtet ist, Sensordatensätze zu speichern, die durch die wenigstens eine Aufprallsensoreinheit (29) erzeugt werden, und/oder ein Ergebnis zu speichern, das durch den Klassifizierungsalgorithmus erhalten wird.

5.  Tragbare medizinische Vorrichtung (1) nach einem der vorhergehenden Ansprüche, wobei der wenigstens eine elektronische Speicher einen separaten elektronischen Aufprallsensordatenspeicher, insbesondere einen Flash-Speicher, umfasst, wobei der separate elektronische Aufprallsensordatenspeicher dazu eingerichtet ist, Aufprallsensordatensätze zu speichern, die durch die wenigstens eine Aufprallsensoreinheit (29) erzeugt werden, und/oder ein Ergebnis zu speichern, das durch den Klassifizierungsalgorithmus erhalten wird.

6.  Tragbare medizinische Vorrichtung (1) nach einem der vorhergehenden Ansprüche, wobei die Vorrichtung (1) ferner eine separate mobile elektrische Leistungsversorgung (21), insbesondere eine Batterie, einen wiederaufladbaren Akkumulator oder einen Kondensator, umfasst, wobei die separate elektrische Leistungsversorgung (21) dazu eingerichtet ist, elektrische Energie an die Aufprallsensoreinheit (29) zu liefern.

7.  Tragbare medizinische Vorrichtung (1) nach einem der vorhergehenden Ansprüche, wobei die Aufprallsensoreinheit (29) dazu eingerichtet ist, eine inaktive mikroelektronische Steuerung zu aktivieren, falls die Aufprallsensoreinheit (29) wenigstens einen Linearbeschleunigungswert ($a_x$; $a_y$; $a_z$) oder wenigstens einen Drehratenwert ($\omega_x$; $\omega_y$; $\omega_z$) oberhalb eines vorbestimmten Schwellenniveaus detektiert.

8.  Tragbare medizinische Vorrichtung (1) nach einem der vorhergehenden Ansprüche, die ferner eine Echtzeituhr umfasst, wobei die Vorrichtung (1) dazu eingerichtet ist, die Zeit eines Aufpralls aufzuzeichnen, falls die Aufprallsensoreinheit (29) wenigstens einen Linearbeschleunigungswert ($a_x$; $a_y$; $a_z$) oder wenigstens einen Drehratenwert ($\omega_x$; $\omega_y$; $\omega_z$) oberhalb eines vorbestimmten Schwellenniveaus detektiert.

9.  Tragbare medizinische Vorrichtung (1) nach einem der vorhergehenden Ansprüche, die ferner eine Echtzeituhr umfasst, wobei die Vorrichtung dazu eingerichtet ist, die Zeit aufzuzeichnen, bei der der Klassifizierungsalgorithmus ein Unterscheidungsergebnis produziert hat.

10. Tragbare medizinische Vorrichtung (1) nach einem der vorhergehenden Ansprüche, wobei die mikroelektronische Steuerung eine Benutzerschnittstelle (27) umfasst, die dazu eingerichtet ist, Benutzereinstellungen zu empfangen und/oder einen oder mehrere Systemzustände anzuzeigen.

11. Tragbare medizinische Vorrichtung (1) nach einem der vorhergehenden Ansprüche, **dadurch gekennzeichnet, dass** die Vorrichtung (1) aus der Gruppe einer ambulanten Wundtherapievorrichtung, insbesondere einer Unterdruckwundtherapievorrichtung (1), einer ambulanten Tiefe-Hirnstimulation-Vorrichtung, eines ambulanten Herzstimulators, einer ambulanten Infusionsvorrichtung, insbesondere einer Insulinpumpe, einer ambulanten Blutdruckeinheit, eines ambulanten Pulsüberwachungsgeräts, einer ambulanten Elektrokardiogrammvorrichtung, einer ambulanten Elektroenzephalogrammvorrichtung und eines ambulanten Diagnosesystems zum Bestimmen von Metaboliten während einer Zirkulation in dem Blut ausgewählt ist.

12. Verfahren zum Unterscheiden eines ersten und eines zweiten Aufprallereignisses, das die folgenden Schritte umfasst:

    - Bereitstellen einer tragbaren medizinischen Vorrichtung (1) nach einem der vorhergehenden Ansprüche,
    - Detektieren des ersten oder eines zweiten Aufprallereignisses mittels der Aufprallsensoreinheit (29),
    - Ausführen eines Klassifizierungsalgorithmus, der es ermöglicht, einen ersten Aufprallsensordatensatz, wobei der erste Aufpralldatensatz mit einem ersten Aufprallereignis korreliert ist, das schädlich für die Vorrichtung (1) ist, von einem zweiten Aufprallsensordatensatz, wobei der zweite Aufpralldatensatz mit einem zweiten Aufprallereignis korreliert ist, das für die Vorrichtung (1) nicht schädlich ist, zu unterscheiden,
    - Aufzeichnen des Ergebnisses, das aus dem Klassifizierungsalgorithmus erhalten wird,
    - optionales Anzeigen einer Alarmnachricht für den Benutzer, falls das Ergebnis, das aus dem Ausführen des Klassifizierungsalgorithmus erhalten wird, angibt, dass ein erstes Aufprallereignis aufgetreten ist, das für die Vorrichtung (1) schädlich ist.

## Revendications

1.  Dispositif médical portable (1), conçu pour être porté par un utilisateur, comprenant :

    - au moins un moyen (5) actionné électriquement pour remplir une fonction thérapeutique et/ou diagnostique chez un patient,
    - au moins une alimentation électrique mobile (21),
    - une unité de commande (18) comportant au moins un dispositif de contrôle microélectronique et au moins une mémoire électronique,

- au moins une unité de capteur d'impact à canaux multiples (29), dans lequel l'unité de capteur d'impact (29) est conçue pour détecter simultanément une accélération linéaire ($a_x$ ; $a_y$ ; $a_z$) le long des trois axes d'espace (x ; y ; z) et des vitesses angulaires ($\omega_x$ ; $\omega_y$ ; $\omega_z$) autour des trois axes d'espace (x ; y ; z), dans lequel l'unité de capteur d'impact (29) génère des ensembles de données de capteur d'impact, et dans lequel les ensembles de données de capteur d'impact sont transférés électroniquement au dispositif de contrôle microélectronique par le biais d'une interface,

- un boîtier (4) dans lequel sont contenus les composants électriques du dispositif (1),

**caractérisé en ce que**

le dispositif de contrôle microélectronique est conçu pour exécuter un algorithme de classification qui permet de discriminer :

- un premier ensemble de données de capteur d'impact, ledit premier ensemble de données d'impact étant corrélé à un premier événement d'impact qui est préjudiciable pour le dispositif (1), et
- un second ensemble de données de capteur d'impact, ledit second ensemble de données d'impact étant corrélé à un second événement d'impact qui n'est pas préjudiciable pour le dispositif (1),

dans lequel l'algorithme de classification comprend une machine à vecteurs de support utilisant un espace à deux dimensions et une ligne de séparation à une dimension (hyperplan « H »), dans lequel la première dimension de l'espace à deux dimensions est définie par la grandeur $M_\alpha$ correspondant à la longueur vectorielle des valeurs d'accélération linéaire ($a_x$ ; $a_y$ ; $a_z$) mesurées par le capteur d'accélération et dans lequel la seconde dimension de l'espace à deux dimensions est définie par la grandeur $M_\omega$ correspondant à la longueur vectorielle des valeurs de vitesse angulaire $\omega_x$ ; $\omega_y$ ; $\omega_z$) mesurées par le capteur de rotation.

2. Dispositif médical portable (1) selon la revendication 1 l'une quelconque des revendications précédentes, dans lequel l'unité de capteur d'impact (29) comprend une unité de mesure inertielle microélectronique (IMU - inertial measurement unit) pour la détection de l'accélération linéaire et de la rotation et dans lequel l'unité de mesure inertielle microélectronique (IMU) est intégrée dans une seule puce.

3. Dispositif médical portable (1) selon l'une quelconque des revendications précédentes, dans lequel la ou les mémoires électroniques sont conçues pour stocker des instructions de fonctionnement pour le dispositif de contrôle microélectronique et/ou pour enregistrer des informations d'état relatives à la fonction du dispositif.

4. Dispositif médical portable (1) selon la revendication 3, dans lequel la ou les mémoires électroniques sont en outre conçues pour stocker des ensembles de données de capteur générés par l'unité ou les unités de capteur d'impact (29) et/ou pour stocker un résultat obtenu par l'algorithme de classification.

5. Dispositif médical portable (1) selon l'une quelconque des revendications précédentes, dans lequel la ou les mémoires électroniques comprennent une mémoire électronique de données de capteur d'impact séparée, en particulier une mémoire flash, dans lequel la mémoire électronique de données de capteur d'impact séparée est conçue pour stocker des ensembles de données de capteur d'impact générés par l'unité ou les unités de capteur d'impact (29) et/ou pour stocker un résultat obtenu par l'algorithme de classification.

6. Dispositif médical portable (1) selon l'une quelconque des revendications précédentes, le dispositif (1) comprenant en outre une alimentation électrique mobile séparée (21), en particulier une batterie, un accumulateur rechargeable ou un condensateur, dans lequel l'alimentation électrique séparée (21) est conçue pour fournir de l'énergie électrique à l'unité de capteur d'impact (29).

7. Dispositif médical portable (1) selon l'une quelconque des revendications précédentes, dans lequel l'unité de capteur d'impact (29) est conçue pour activer un dispositif de contrôle microélectronique inactif, si l'unité de capteur d'impact (29) détecte au moins une valeur d'accélération linéaire ($a_x$ ; $a_y$ ; $a_z$) ou au moins une valeur de vitesse angulaire $\omega_x$ ; $\omega_y$ ; $\omega_z$) au-dessus d'un niveau seuil prédéterminé.

8. Dispositif médical portable (1) selon l'une quelconque des revendications précédentes, comprenant en outre une horloge en temps réel, par laquelle le dispositif (1) est conçu pour enregistrer l'heure d'un impact, si l'unité de capteur d'impact (29) détecte au moins une valeur d'accélération linéaire ($a_x$ ; $a_y$ ; $a_z$) ou au moins une valeur de vitesse angulaire $\omega_x$ ; $\omega_y$ ; $\omega_z$) au-dessus d'un niveau seuil prédéterminé.

9. Dispositif médical portable (1) selon l'une quelconque des revendications précédentes, comprenant en outre une horloge en temps réel, par laquelle le dispositif est conçu pour enregistrer l'heure à laquelle l'algorithme de classification a produit un résultat de discrimination.

10. Dispositif médical portable (1) selon l'une quelconque des revendications précédentes, dans lequel le dispositif de contrôle microélectronique comprend une interface d'utilisateur (27) adaptée pour recevoir des paramètres d'utilisateur et/ou pour afficher une ou plusieurs conditions de système.

11. Dispositif médical portable (1) selon l'une quelconque des revendications précédentes, **caractérisé en ce que** le dispositif (1) est choisi dans le groupe constitué par un dispositif ambulatoire de traitement de plaies, en particulier un dispositif de traitement de plaies par pression négative (1), un dispositif ambulatoire de stimulation cérébrale profonde, un stimulateur cardiaque ambulatoire, un dispositif de perfusion ambulatoire, en particulier une pompe à insuline, une unité ambulatoire de pression artérielle, un moniteur ambulatoire du pouls, un dispositif ambulatoire pour électrocardiogramme, un dispositif ambulatoire pour électroencéphalogramme et un système de diagnostic ambulatoire pour déterminer les métabolites circulant dans le sang.

12. Procédé de discrimination d'un premier et d'un second événement d'impact, comprenant les étapes consistant à :

> - fournir un dispositif médical portable (1) selon l'une quelconque des revendications précédentes,
> - détecter le premier ou le second événement d'impact au moyen de l'unité de capteur d'impact (29),
> - exécuter un algorithme de classification permettant de discriminer un premier ensemble de données de capteur d'impact, ledit premier ensemble de données d'impact étant corrélé à un premier événement d'impact préjudiciable pour le dispositif (1) par rapport à un second ensemble de données de capteur d'impact, ledit second ensemble de données d'impact étant corrélé à un second événement d'impact qui n'est pas préjudiciable pour le dispositif (1),
> - enregistrer le résultat obtenu à partir de l'algorithme de classification,
> - éventuellement afficher un message d'alarme à l'utilisateur, si le résultat obtenu de l'exécution de l'algorithme de classification indique qu'un premier événement d'impact qui est préjudiciable pour le dispositif (1) s'est produit.

FIG 1

FIG 2

Fig. 2a

Fig. 2b

Fig. 2c

Fig. 2d

Fig. 2e

FIG 3

FIG 4

EP 3 187 201 B2

Control unit

FIG 5

FIG 6

FIG 7 a

FIG 7 b

FIG 7 c

Magnitude of acceleration signals

Magnitude of turnrate signals

FIG 7 d

Magnitude of acceleration signals

Magnitude of turnrate signals

FIG 7 e

Magnitude of acceleration signals

Magnitude of turnrate signals

FIG 7 f

Magnitude of acceleration signals

Magnitude of turnrate signals

FIG 8 a

FIG 8 b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150040665 A1
- US 20150073338 A1
- EP 2345893 A1
- US 20040073151 A1
- WO 2009047524 A2
- EP 1905465 A1
- WO 2008039314 A2
- EP 77750481 A
- EP 1863549 B1
- EP 2464394 A1
- WO 2012156174 A1
- EP 2464393 A1
- US 20080276684 A
- EP 1867955 A